# FASCICULE DE BREVET EUROPEEN

(11) **EP 0 758 383 B1**
(45) Date de publication et mention de la délivrance du brevet: **24.01.2007**
(21) Numéro de dépôt: 95920125.2
(22) Date de dépôt: 05.05.1995
(51) Int. Cl.: C12N 15/12, C07K 14/705, A61K 38/17, A61K 39/395, C07K 16/42

(54) **FRACTIONS POLYPEPTIDIQUES SOLUBLES DE LA PROTEINE LAG-3; PROCEDE DE PRODUCTION; COMPOSITION THERAPEUTIQUE; ANTICORPS ANTI-IDIOTYPE**
LÖSLICHE POLYPEPTIDFRAKTIONEN DES LAG-3-PROTEINS; VERFAHREN ZUR HERSTELLUNG; THERAPEUTISCHE ZUSAMENSETZUNG; ANTIIDIOTYPISCHER ANTIKÖRPER
LAG-3 PROTEIN SOLUBLE POLYPEPTIDE FRACTIONS, METHOD OF PRODUCTION, THERAPEUTIC COMPOSITION AND ANTI-IDIOTYPE ANTIBODY

(30) Priorité: 06.05.1994 FR 9405643
(43) Date de publication de la demande: 19.02.1997
(73) Titulaire: Institut Gustave Roussy, 94805 Villejuif Cédex (FR); INSTITUT NATIONAL DE LA SANTE ET DE LA RECHERCHE MEDICALE (INSERM), 75013 Paris (FR); Applied Research Systems ARS Holding N.V., Curaçao (AN)
(72) Inventeur: FAURE, Florence, F-75016 Paris (FR); HERCEND, Thierry, F-94220 Charenton-le-Pont (FR); HUARD, Bertrand, F-94240 L'Haye-les-Roses (FR); TRIEBEL, Frédéric, F-78000 Versailles (FR)
(74) Mandataire: Breese, Pierre
(86) Numéro de dépôt international: PCT/FR1995/000593
(87) Numéro de publication internationale: WO 1995/030750

(56) Documents cités:
- WO-A-91/10682
- WO-A-92/00092
- THE JOURNAL OF EXPERIMENTAL MEDICINE, vol. 176, no. 2, Août 1992 NEW YORK NY, TATS UNIS, pages 327-337, E. BAIXERAS ET AL. 'Characterization of the lymphocyte activation gene 3-encoded protein. A new ligand for human leukocyte antigen class II antigens.' cité dans la demande
- THE JOURNAL OF EXPERIMENTAL MEDICINE, vol. 171, no. 5, Mai 1990 NEW YORK NY, TATS UNIS, pages 1393-1405, F. TRIEBEL ET AL. 'LAG-3, a novel lymphocyte activation gene closely related to CD4.' cité dans la demande
- B. HUARD et al. In: "8 International Congr of Immunology. Abstracts". Budapest Hungar Aug. 23-28, 1992. Springer Verlag, Budapes 1992, p. 281.
- IMMUNOGENETICS, vol. 39, no. 3, Mars 1994 NEW YORK NY, TATS UNIS, pages 213-217, B. HUARD ET AL. 'Cellular expression and tissue distribution of the human LAG-3-encoded protein, an MHC class II ligand.'
- JOURNAL OF CELLULAR BIOCHEMISTRY, SUPPLEMENT, vol. 0, no. 21a, 16 Mars 1995 - 22 Mars 1995 NEW YORK, NY, ¹TATS UNIS, page 120 B. HUARD ET AL. 'LAG-3/MHC class II interaction modulates the antigenic response of CD4+ T cell clones.'
- MAÇON-LEMAÎTRE L.; TRIEBEL F.: 'The negative regulatory function of the lymphocyte-activation gene-3 co-receptor (CD223) on human T cells' IMMUNOLOGY vol. 115, 2005, pages 170 - 178
- FARACE F. ET AL: 'Metastatic renal-cell carcinoma patients treated with interleukin 2 or interleukin 2 plus interferon gamma:immunological monitoring' INT. J. CANCER vol. 57, 1994, pages 814 - 821
- WORKMAN C.J.; VIGNALI D.A.A.: 'Negative regulation of T cell homeostasis by Lymphocyte Activation gene-3 (CD223)' THE JOURNAL OF IMMUNOLOGY vol. 174, 2005, pages 688 - 695

## Description

L'invention concerne des formes solubles dérivées de la protéine membranaire LAG-3 utiles en tant qu'immunodépresseurs ainsi que des anticorps susceptibles d'empêcher la fixation spécifique de la protéine LAG-3 à des molécules du CMH (Complexe majeur d'histocompatibilité) de classe II en tant qu'immunostimulants.

Dans WO-A 91/10682, on a décrit une protéine désignée LAG-3.

La protéine LAG-3 est une protéine sélectivement exprimée par les cellules NK et les lymphocytes T activés. La similitude de la séquence d'aminoacides, l'organisation comparée exon/intron et la localisation chromosomique montrent que LAG-3 est apparenté à CD4. La caractérisation initiale du gène de LAG-3 a été décrite par TRIEBEL et al. (1).

L'ADN correspondant code pour une protéine transmembranaire de 498 aminoacides de type I comportant 4 séquences extracellulaires de type immunoglobuline. LAG-3 est un membre de la super-famille des immunoglobulines.

La protéine mature comprend 476 aminoacides (SEQ ID N° 1) avec un poids moléculaire théorique de 52 kD. La région extracellulaire contient 8 résidus cystéine et 4 sites de N-glycosylation potentiels. Par analyse par Western-Blot, on a montré que LAG-3 à l'intérieur des PHA-blastes ou des cellules NK activées a une masse apparente Mr de 70 000. Après traitement par la N-glycosydase F, une réduction de taille à 60 kD était obtenue, démontrant ainsi que LAG-3 native est glycosylée. De plus amples détails sont décrits dans WO-A 91/10682.

BAIXERAS et al., dans J. Exp. Med. 176, 327-337 (2), ont en outre décrit que la formation de rosettes entre des cellules transfectées par LAG-3 (exprimant LAG-3 à leur surface) et des lymphocytes B exprimant le CMH de Classe II était dépendante de façon spécifique de l'interaction LAG-3 / CMH de Classe II.

De manière surprenante, ce ligand du CMH de Classe II a été détecté avec des taux plus élevés sur des lymphocytes CD8⁺ (CMH Classe I-restreints) activés que sur des lymphocytes CD4⁺ activés. In vivo, seules quelques cellules LAG-3⁺ (CMH Classe II-restreints) disséminées ont été retrouvées dans du tissu lymphoïde non hyperplasique comprenant les organes lymphoïdes primaire, c'est-à-dire le thymus et la moelle osseuse. Des cellules LAG-3⁺ ont été retrouvées dans des nodules lymphoïdes hyperplasiques et des amygdales, ainsi que sur des cellules mononucléées de sang périphérique (PBMC) de patients recevant des injections de doses élevées d'IL-2.

Ces observations confirment que LAG-3 est un antigène d'activation par contraste avec CD4 exprimé dans une sous-population de lymphocytes au repos et d'autres types cellulaires, notamment les macrophages.

Le CMH comprend les molécules de Classe I et de Classe II qui sont des glycoprotéines membranaires qui présentent des fragments d'antigènes protéiques aux récepteurs des lymphocytes T (TCR). Les molécules de Classe I sont responsables de la présentation aux cellules cytotoxiques CD8⁺ de peptides dérivant en grande partie de protéines synthétisées de façon endogène tandis que les molécules de Classe II présentent aux lymphocytes auxilliaires CD4⁺ des peptides provenant en premier lieu de protéines étrangères qui sont entrées dans la voie endocytique, c'est-à-dire exogène. Les lymphocytes T auxilliaires régulent et amplifient la réponse immunitaire, tandis que les lymphocytes cytotoxiques sont nécessaires pour détruire les cellules, quels que soient les tissus exprimant les antigènes du "non-soi", par exemple les antigènes viraux. Le mécanisme de la reconnaissance fait intervenir des signaux intracellulaires conduisant à une activité effective des lymphocytes T.

Il apparaît que pour initier une réponse immunitaire à médiation de lymphocytes T (CD4⁻), les antigènes étrangers doivent être capturés, internalisés sous forme de peptides par des cellules spécialisées, les cellules présentant l'antigène (APC). Les peptides antigèniques résultant sont re-exprimés à la surface des cellules de présentation de l'antigène où ils sont associés avec les molécules du CMH de Classe II. Ce complexe CMH de Classe II / peptide est spécifiquement reconnu par le récepteur du lymphocyte T, d'où il résulte une activation des lymphocytes T auxilliaires.

Par ailleurs, des modèles animaux créés par des techniques de recombinaison ont permis de souligner le rôle joué in vivo par les molécules du CMH de Classe II et leurs ligands.

Ainsi, des souris déficientes en molécules du CMH de Classe II (3) et ne possédant pratiquement pas de lymphocytes T CD4⁺ périphériques et n'ayant que quelques lymphocytes CD4⁺ immatures dans le thymus se sont révélées être totalement inaptes à répondre aux antigènes T-dépendantes.

Les souris mutantes CD4⁻/⁻ (4) ont une activité de lymphocytes T sensiblement diminuée mais montrent un développement et une fonction normaux des lymphocytes T CD8⁺, ce qui démontre que l'expression de CD4 sur les cellules filles et les thymocytes CD4⁺ CD8⁺ n'est pas obligatoire pour le développement. Comparées à des souris normales, des souris déficientes en CD4 ont une grande quantité de cellules CD4⁻ CD8⁻.

Ces cellules doublement négatives sont restreintes au CMH de Classe II et capables de reconnaître l'antigène.

Lorsqu'elles sont infectées par des leishmania, ces souris montrent une population de lymphocytes T auxilliaires fonctionnels malgré l'absence de CD4. Ces cellules sont restrictives au CMH de Classe II et produisent de l'interferon-γ lorsqu'elles sont activées par l'antigène. ceci indique que le lignage des lymphocytes T et leur fonction périphérique ne doit pas nécessairement dépendre de la fonction de CD4.

Il est maintenant reconnu que les protéines codées par la région du CMH de Classe II sont impliquées dans de nombreux aspects de la reconnaissance immunitaire, y compris l'interaction entre différentes cellules lymphoïdes, comme les lymphocytes et les cellules de présentation de l'antigène. Différentes observations ont également montré que d'autres mécanismes qui n'ont pas lieu par l'intermédiaire de CD4 interviennent dans la fonction effectrice des lymphocytes T auxilliaires.

Ces différentes observations soulignent le rôle pivot joué par la CMH de Classe II et ses ligands dans le système immunitiare.

Par ailleurs, on connaît l'intérêt des molécules chimères composées du domaine extra-cytoplasmique de protéines capables de se fixer à des ligands et une région constante des chaînes d'immunoglobulines (Ig) humaines pour l'obtention de formes solubles de protéines et de récepteurs cellulaires utiles, notamment comme agents thérapeutiques.

C'est ainsi que des formes solubles de CD4 ont fait la preuve de leur efficacité à inhiber une infection par le VIH in vitro d'une manière dose-dépendante.

Néanmoins, des essais cliniques avec des molécules de CD4 solubles, notamment de CD4-Ig n'ont pas permis de mettre en évidence de diminution significative des titres viraux. Des souris transgéniques exprimant jusqu'à 20 µg/ml de CD4 solubles dans leur sérum ont été créées. Ces souris n'ont montré aucune différence en ce qui concerne leur fonction immunitaire par rapport à des souris témoins. Jusqu'à présent, aucune liaison directe au CMH de Classe II de molécules dérivant de CD4 n'a été reportée. Ceci suggère fortement que les CD4 solubles n'interagissent pas in vivo avec les molécules du CMH de Classe II.

De manière surprenante, les auteurs de la présente invention ont montré que des molécules solubles contenant différents fragments du domaine extracytoplasmique de la protéine LAG-3 étaient capables de se lier aux molécules du CMH de Classe II et d'avoir une action immunosuppressive.

La région extracytoplasmique de LAG-3 représentée par la séquence SEQ ID N° 1 comprend les domaines D1, D2, D3, D4 s'étendant des aminoacides 1 à ±49, 150 à 239, 240 à 330 et 331 à 412, respectivement.

L'invention a ainsi pour objet une fraction polypeptidique soluble constituée par les 4 domaines extracellulaires de type immunoglobuline de la protéine LAG-3 (aminoacide 1 à 149, 150 à 239, 240 à 330 et 331 à 412 de la séquence SEQ ID N° 1) et éventuellement une séquence peptidique supplémentaire à son extrémité C-terminale et/ou N-terminale, de manière à constituer une protéine de fusion. Le terme "protéine de fusion" signifie une partie d'une protéine quelconque permettant la modification des caractéristiques physico-chimiques des sous-fragments du domaine extracytoplasmique de la protéine LAG-3. Des exemples de telles protéines de fusion contiennent des fragments du domaine extracytoplasmique de LAG-3 tels que définis ci-dessus liés à la région jonction -CH2-CH3 de la chaîne lourde d'une immunoglobuline humaine, de préférence une immunoglobuline d'isotype IgG4.

De telles protéines de fusion peuvent être dimériques ou monomériques. Ces protéines de fusion peuvent être obtenues par des techniques de recombinaison bien connues de l'homme du métier, par exemple une technique telle que décrite par Traunecker et al. (5).

De manière générale, le procédé de production de ces protéines de fusion comprenant une région d'immunoglobuline fusionnée avec une séquence peptidique de LAG-3 telle que définie ci-dessus, consiste en ce que l'on insère dans un vecteur les fragments d'ADNc codant pour les régions polypeptidiques correspondant à LAG-3 ou dérivées de LAG-3, éventuellement après amplification par PCR et l'ADNc codant pour la région pertinente de l'immunoglobuline, fusionné avec l'ADNc codant pour les régions polypeptidiques correspondantes ou dérivés de LAG-3, et que l'on exprime après transfection, les fragments de l'ADNc dans un système d'expression, notamment des cellules de mammifères, par exemple des cellules d'ovaire de hamster.

Les protéines de fusion selon l'invention peuvent également être obtenues par clivage d'un conjugué LAG-3 Ig construit de manière à contenir un site de clivage approprié.

L'invention a également pour objet une composition thérapeutique d'activité immuno-suppressive comprenant une fraction polypeptidique soluble selon l'invention. Cette composition sera utile pour traiter des pathologies nécessitant une immuno-suppression, par exemple les maladies auto-immunes.

L'invention a également pour objet l'utilisation d'anticorps reconnaissant le domaine extracellulaire D1 de type immunoglobuline de la protéine LAG-3 ou des fragments de tels anticorps, notamment les fragments Fab, Fab', F(ab')₂ pour la préparation d'une composition thérapeutique à activité immunostimulante. Le terme "immunostimulant" signifie une entité moléculaire capable de stimuler la maturation, la différenciation, la prolifération et/ou la fonction de cellules exprimant LAG-3, c'est-à-dire de lymphocytes T ou cellules NK actives. Les anticorps anti-LAG-3 peuvent être utilisés comme potentialisateurs de vaccins ou immunostimulants chez des patients immunodéprimés, tels les malades infectés par le VIH ou traités par des substances immunosuppressives, ou être utilisés pour stimuler le système immunitaire par élimination de cellules du soi présentant un comportement anormal, par exemple des cellules cancéreuses.

L'activité immunostimulante des anticorps anti-LAG-3 est surprenante dans la mesure où les anticorps anti-CD4 ont une action immuno-suppressive.

De tels anticorps peuvent être polyclonaux ou monoclonaux ; cependant, on préfère les anticorps monoclonaux. Les anticorps polyclonaux peuvent être préparés selon des méthodes bien connues, telle celle décrite par BENEDICT A.A. et al. (6). On préfère les anticorps monoclonaux, du fait qu'ils sont spécifiques d'un épitope unique et fournissent des résultats avec une reproductibilité meilleure. Des méthodes de production d'anticorps monoclonaux sont bien connues de l'état de la technique, en particulier celle décrite par KOHLER et MILSTEIN. Cette methode, ainsi que des variantes de celle-ci, sont décrites par YELTON et al. (7).

L'invention a également pour objet des anticorps anti-idiotype dirigés contre les anticorps selon l'invention, qui comportent l'image interne de LAG-3 et sont par conséquent apte à se lier au CMH de Classe II. De tels anticorps peuvent être utilisés notamment comme immunodépresseurs, et par exemple dans des pathologies auto-immunes.

Les compositions thérapeutiques selon la présente invention comprennent des protéines LAG-3 solubles ou des anticorps tels que définis ci-dessus, ainsi qu'un véhicule pharmaceutiquement acceptable. Ces compositions peuvent être formulées selon les techniques habituelles. Le véhicule peut être de forme variée en fonction de la voir d'administration choisie : orale, parentérale, sublinguale, rectale, ou nasale.

Pour les compositions à administration parentérale, le véhicule comprendra généralement de l'eau stérile ainsi que d'autres ingrédients éventuels promouvant la solubilité ou l'aptitude à la conservation de la composition. Les voies d'administration parentérales peuvent consister en injections intraveineuses, intramusculaires ou sous-cutanées.

La composition thérapeutique peut être à libération prolongée, notamment pour les traitements à long terme, par exemple dans les maladies auto-immunes. La dose à administrer dépend du sujet à traiter, notamment de la capacité de son système immunitaire à atteindre le degré de protection souhaité. Les quantités précises d'ingrédient actif à administrer peuvent être déterminés sans effort par le praticien qui initiera le traitement.

Les compositions thérapeutiques selon l'invention peuvent comprendre en plus de LAG-3 soluble ou des anticorps selon l'invention un autre ingrédient actif, éventuellement lié par une liaison chimique à LAG-3 ou à un anticorps selon l'invention. On citera à titre d'exemple des protéines LAG-3 solubles selon l'invention fusionnées à une toxine : par exemple la ricine ou l'anatoxine diphtérique, susceptibles de se fixer à des molécules de CMH de Classe II et de tuer les cellules cibles, par exemple des cellules leucémiques ou de mélanome, ou fusionnées à un radioisotope.

Les exemples suivants, ainsi que les figures de références annexées, illustreront plus en détail l'invention.

### EXEMPLE 1

### Prolifération de lignées de lymphocytes T actives en présence d'anticorps monoclonaux anti-LAG-3

Les anticorps monoclonaux anti LAG-3 utilisés étaient 17 B4, décrit dans BAIXERAS et al. (2) et déposé à la CNCM sous le n° I-1240 le 10 Juillet 1992, et 11 E3, décrit dans HUARD et al. (8). L'anticorps 17B4 reconnaît spécifiquement le domaine extracellulaire D1 de type immunoglobuline de la protéine LAG-3 tandis que l'anticorps 11E3 reconnaît le domaine extracellulaire D3.

Ces anticorps appartiennent à l'isotype IgG1. Ces anticorps ont été testés pour leurs effets biologiques sur des lymphocytes T activés, stimulés par des peptides antigèniques spécifiques ou des antigènes remaniés présentés par des molécules du CMH de Classe II exprimées par des cellules de présentation de l'antigène autologues, exprimant LAG-3.

Un anticorps monoclonal anti-CD48 dénommé 10 H3 a été utilisé comme anticorps IgG1 non pertinent (témoin négatif).

Les concentrations saturantes d'anticorps anti-LAG-3 et anti-CD48 ont été déterminées par immunofluorescence sur des blastes-PHA (phytohémaglutinine) et des lignées cellulaires transformées par le virus d'Epstein Barr (EBV). Dans les essais de prolifération, les anticorps monoclonaux ont été ajoutés à raison de 5 fois la concentration saturante.

Les lignées de lymphocytes T utilisées étaient d'une part le clone 154 dérivé de lymphocytes de sang périphérique éduqué contre un peptide mimiquant un fragment de l'hémaglutinine (HA) d'influenza ayant une séquence d'aminoacide s'étendant de l'aminoacide 306 à 329 (peptide p20), d'autre part, le clone 28, un clone de lymphocyte T dérivé de lymphocytes périphérique d'un seul donneur humain éduqués contre l'anatoxine diphtérique (DT). Les cellules de présentation del'antigène (APC) correspondant au clone 154 étaient des lymphocytes B transformés par l'EBV du même donneur (DR3/DR11) que T 154. Les cellules de présentation de l'antigène correspondant au clone 28 étaient des lymphocytes B transformés par l'EBV du même donneur. Ce clone était restreint à HLA DR7.

Pour le clone 154, les APC (5 x 10⁶) ont été mises à incuber à 37°C pendant 1 heure et demi avec des doses variables du peptide p20, puis lavées et irradiées (10 000 rad.). Les cellules ont été étalées sur des plaques de microtitration à 96 puits en même temps que les cellules du clone 154 (0,5 x 10⁵ à 10 x 10⁵cell/ml) dans un rapport 3/1. Pour le clone 28, le rapport cellules répondantes/cellules stiumlantes était de 1.

Les cellules APC HLA DR7/EBV étaient soit traitées à la mitomycine ou irradiées, puis ajoutées aux lymphocytes T en présence de DT (qui restait dans la culture). La concentration finale en cellules du clone 28 était de 100 000 cell./ml.

De la ³H-thymidine (1 µCi/puit) était ajoutée à des intervalles de temps variés à partir du jour 2 jusqu'au jour 10 de culture.

Chaque expérience a été réalisée en trois exemplaires.

Les résultats ont été exprimés comme moyenne des cpm et après soustraction des cpm trouvés dans le témoin négatif (lymphocytes T co-cultivées avec des APC non chargés en immunogènes). Les essais de prolifération ont été réalisés sur des plaques à 96 puits. L'absorption de thymidine tritiée des puits individuels de 200 µl a été mesurée après addition de 1 µCi de thymidine pour les dernières 18 heures de culture. Les résultats ont été exprimés sous forme de moyenne de 3 essais. L'écart type était habituellement inférieur à 12% (un peu plus pour les mesures de cpm très faibles). D'autre part, des surnageants de culture mixte (clone 154/APC) ont été rassemblés, filtrés sur des membranes de 0,22 pm, divisés en échantillons et congelés à -20°C jusqu'au moment de la titration à l'aide de kits d'immuno-essais du commerce : kit de titration IL-2 et INF-α d'Immunotech, kit IFN-γ de Genzyme et kit IL-4 de Cayman Chemicals.

Une étude de détermination de la dose a été réalisée pour établir les profils de prolifération du clone 154 mis en présence de l'antigène spécifique p20 à des concentrations variées et en présence ou non des anticorps monoclonaux anti-LAG-3 ou des anticorps monoclonaux non pertinents (témoin négatif).

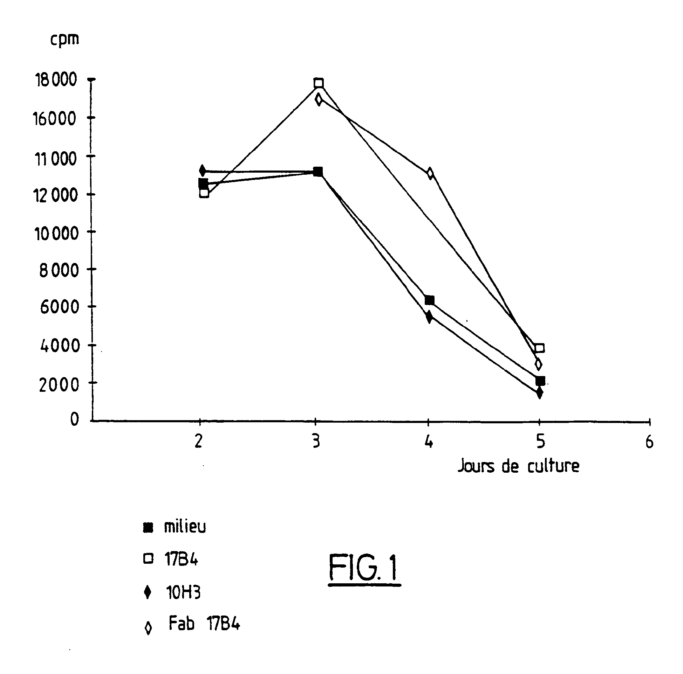

Les résultats individuels de 16 essais distincts ont montré que, quelle que soit la concentration en antigène ajouté, le point initial jusqu'au pic de prolifération n'était pas modifié, mais une prolongation significative de la prolifération de lymphocytes T incubés avec les anticorps monoclonaux anti-LAG-3 était observée de façon systématique. Des fragments Fab de l'anticorps monoclonal 17B4 ont été préparés et utilisés dans un essai de prolifération du clone 154. Le profil de prolifération des lymphocytes T activés par l'antigène avec les fragments Fab 17B4 (15 µg/ml) était similaire à celui de cellules incubées en présence de l'anticorps monoclonal 17B4 entier (40 µg/ml) (Figure 1). Ces résultats montrent que les effets biologiques observés ne sont pas imputables à une réaction non spécifique induite par la région Fc des anticorps monoclonaux anti-LAG-3.

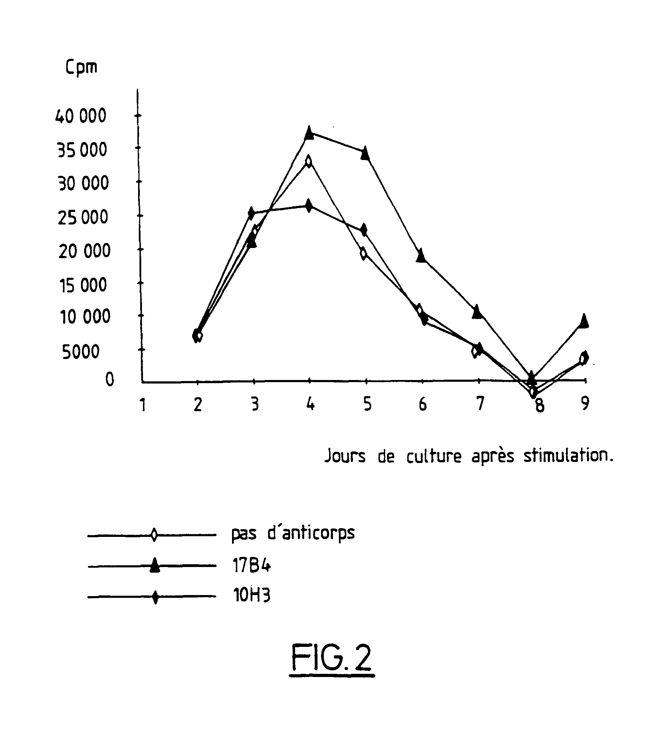

Le clone 28 a également été stimulé par l'antigène (anatoxine tétanique 10 mcg/ml) en présence des anticorps monoclonaux 17B4 après co-culture avec les APC correspondants en présence de DT. Les résultats sont représentés à la figure 2.

Les effets des anticorps monoclonaux anti-LAG-3 observés avec le clone 28, à savoir la prolongation de la prolifération sont similaires à ceux observés avec le clone 154.

Des essais destinés à mesurer les évènements cellulaires variés se produisant après la stimulation antigénique des cellules du clone 154 incubées en présence d'anticorps monoclonaux anti-LAG-3 ont été réalisés.

Les cellules ont été récoltées pendant la stimulation antigénique classique du clone 154 en présence d'anticorps monoclonaux anti-LAG-3 ou anti-CD48 ou en l'absence d'anticorps et testées pour l'expression des récepteurs transmembranaires LAG-3 et CD25 et des échantillons de surnageants de culture ont été collectés à différents intervalles de temps après la stimulation et testés pour la présence d'IFN-γ, TNF-α, IL-4 et IL-2.

Des essais d'immunofluorescence directe à 2 couleurs (anticorps monoclonaux anti-CD3 et anticorps monoclonaux anti-CD 25) ont montré que les récepteurs pour l'IL-2 étaient faiblement mais significativement augmentés 5 jours après la stimulation antigénique. Des essais similaires avec les anticorps monoclonaux anti-CD3 et 11E3 (anti-LAG-3) ont montré que LAG-3 était sur-exprimée dès le jour suivant l'activation. En outre, la sécrétion d' IL-2, IL-4, IFN-γ et de TNF-α était également modulée par l'incubation avec des anticorps monoclonaux anti-LAG-3, montrant ainsi que différents évènements cellulaires sont modifiés par la présence d'anticorps monoclonaux anti-LAG-3 et que certains évènements ont déjà lieu 24 heures après la stimulation.

Ces résultats montrent indirectement que LAG-3 joue un rôle régulateur pour les cellules CD4⁺. Le fait que les anticorps monoclonaux anti-LAG-3 augmentent la prolifération, donc agissent comme des immunopotentialisateurs, suggère que LAG-3 est impliqué dans la "désactivation" de lymphocytes T CD4⁺ avec un rôle négatif de LAG-3 sur la stimulation dépendante de l'antigène.

### EXEMPLE 2

### Expression transitoire de protéines de fusion LAG-3

Des protéines solubles dérivant de LAG-3 ont été obtenues par une technique d'ADN recombinant à l'aide de vecteurs appropriés comprenant l'ADN codant pour LAG-3 et l'ADN codant pour un fragment d'immunoglobuline. Le système d'expression transitoire consistait en cellules Cos transfectées. Ce système rend possible la production de plusieurs mg de protéines de fusion recombinantes. Les techniques de l'ADN recombinant ont été mises en oeuvre comme décrit par MANIATIS et al. (22). Les modifications ont été faites comme recommandé par le fabricant.

### Construction de LAG-3 D1-D4 Ig et LAG-3 D1D2 Ig

Des fragments codant pour les régions D1D2 ou D1-D4 ont été amplifiés (30 cycles) à partir d'un fragment d'ADNc (séquence FDC) englobant l'ADNc LAG-3 (TRIEBEL et al. (1)) à l'aide de la polymérase Taq exempte d'activité de 5'-endonucléase et relativement résistante à une exposition à une température très élevée ; l'amplification était suivie d'une dénaturation à 98°C (avec un "DNA thermal cycle Perkin Elmer Cetus"). Des amorces spécifiques ont été utilisées comme rapporté au tableau ci-dessous.

Les fragments amplifiés résultant (739 pb et 1312 pb pour LAG-3 D1D2 et LAG-3 D1-D4, respectivement) ont été insérés dans un plasmide pBS (Stratagène).

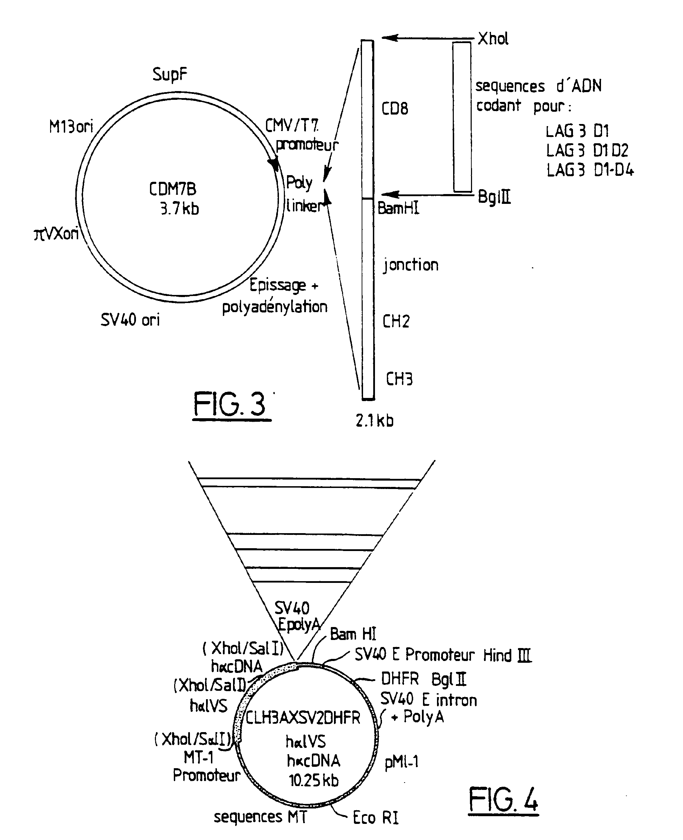

Des inserts ont été préparés après digestion avec XhoI et Bgl II et introduits dans les sites Xho I/Bam HI du vecteur p CDM7-CD8-IgG1 (p CDM7 étant dérivé de p CDM8 commercialisé par Stratagène), comme illustré sur la figure 3, afin d'échanger les séquences d'ADN codant pour CD8 par celles codant, pour les sous-fragments de LAG-3. Les vecteurs d'expression résultant contenaient les séquences codant pour D1D2 ou D1-D4 fusionnées aux séquences d'ADN codant pour la région jonction -CH2-CH3 d'une chaîne d'IgGl humaine.

**TABLEAU 3**

| **Amorces utilisées pour amplifier des séquences d'ADN de LAG-3 par PCR** | | | |
|---|---|---|---|
| **Amorces utilisées pour l'amplification de l'ADN** | | | **Sous-fragment codé résultant fusionné avec un sous-fragment d'Ig** |
| Amorce (5') | | | LAG-3 D1D2 |
| 5' GCGCCTCGAGGCCCAGACCATAGGAGAGATGT 3' | | | |
| site d'accrochage | 5' séquences non traduites | début de traduction | depuis la séquence leader jusqu'à l'amino-acide 241 |
| Amorce (3') | | | |
| 5' GCGCAGATCTCTCCAGACCCAGAACAGTGAGGTTATACAT 3' | | | |
| Site | | Fin de D2 | |
| d'accrochage Bgl II | | | |
| Amorce (5') | | | LAG-3 D1-D4 |
| identique à LAG-3 D1D2 | | | |
| Amorce (3') | | | depuis la séquence leader jusqu'à l'amino-acide 412 |
| 5' GCGCAGATCTACCTGGGCTAGACAGCTCTGTGAA 3' | | | |
| Site | | Fin de D4 | |
| d'accrochage Bgl II | | | |

CDM7 est un vecteur d'expression eucaryote dérivé des vecteurs développés par SEED et al. (10) pour le clonage d'ADN et son expression dans *E. coli* et des cellules eucaryotes. CDM7 possède les caractéristiques suivantes : (i) le promoteur du cytomégalovirus humain pour l'expression transitoire dans des cellules de mammifères ; (ii) une origine virale de SV40 pour une replication autosomale des cellules de mammifères exprimant l'antigène T ; (iii) n VX (type Col E1) comme origine plasmidique pour un nombre élevé de copies ; (iv) une sélection Sup F pour la résistance à l'ampicilline et la tétracycline dans des souches D'*E. coli* Tet^{amb} et Amp^{amb} ; (v) une origine de replication de M13 pour la libération d'un simple brin ; (vi) un promoteur à ARN de T7 ; et (vii) un polylinker pour un clonage efficace d'ADN hétérologue.

### Expression transitoire dans des cellules Cos

Des cellules Cos (5 x 10⁶) ont été transfectées avec 30 µg d'ADN de vecteurs d'expression appropriés (codant, soit pour LAG-3 D1D2 Ig, soit LAG-3 D1-D4 Ig, soit CD8 Ig) par électroporation (200 V, 1500 µF, 30-40 msec) à l'aide d'un appareil Cellject (Eurogentech, Liège, BE). Les cellules ont été ré-étalées et cultivées sur un milieu contenant 5% de sérum foetal de veau. Les surnageants ont été prélevés 6 jours après la transfection.

La synthèse des protéines de fusion résultante a été analysée à partir des surnageants, ainsi que des extraits cellulaires de cellules transfectées par analyse de Western-Blot avec les anticorps monoclonaux 17B4. Les matériaux immunoréactifs ont été observés dans le surnageant de cellules transfectées avec de l'ADN codant pour LAG-3 D1D2 Ig ou LAG-3 D1-D4 Ig.

Parallèlement, une immunoadhésine CD8 (CD8 Ig) recombinante a été obtenue comme témoin négatif à l'aide du même système d'expression et du vecteur d'expression pCDM7-CD8 (Figure 3).

Les protéines recombinantes LAG-3 D1D2 Ig, LAG-3 D1-D4 Ig et CD8 Ig ont été purifiées grâce à la méthode classique sur Protéine A-Sépharose. Le matériau résultant a été analysé par SDS-PAGE, suivi par une coloration de Coomassie ou une analyse de Western-Blot à l'aide d'anticorps anti-Ig humaine.

### EXEMPLE 3

### Production de sous-fragments solubles de LAG-3

Afin de produire de grandes quantités de protéines recombinantes, un système d'expression stable consistant en cellules de mammifères transfectées a été développé. Les cellules-hôtes sont des cellules d'ovaire de hamster (CHO) dépendantes d'un ancrage, isolées à partir de cellules CHO déficientes en dihydrofolate réductase (dhfr) et nécessitant par conséquent de la glycine, une purine et de la thymidine pour leur croissance. Le rôle pivot de la dhfr dans la synthèse de précurseurs nucléiques, combiné avec la sensibilité des cellules dhfr déficientes à l'égard des analogues du tétrahydrofolate tels que le méthotrexate (MTX) présente deux avantages importants. La transfection de ces cellules avec des vecteurs d'expression contenant le gène dhfr permet la secrétion de clones dhfr-résistants recombinants et la culture de ces cellules sur des milieux sélectifs contenant des quantités croissantes de MTX résulte en l'amplification du gène dhfr et l'ADN qui y est associé.

### Construction de LAG-3 D1, LAG-3 D1D2, LAG-3 D1-D4

Des fragments d'ADN codant pour les régions D1, D1D2 ou D1-D4 ont été amplifiés à l'aide d'un procédé de PCR identique à celui décrit précédemment à l'aide des amorces précisées dans le Tableau ci-dessous.

**TABLEAU 4**

| **Amorces utilisées pour amplifier des séquences d'ADN de LAG-3 par PCR** | | | |
|---|---|---|---|
| **Amorces utilisées pour l'amplification de l'ADN** | | | **Sous-fragment codé résultant** |
| Amorce (5') | | | LAG-3 D1 |
| 5' CGCCGTCGACCGCTGCCCAGACCATAGGAGAGATGTG 3' | | | |
| | | | depuis la |
| Site | 5' séquences | Début de | séquence leader |
| d'accrochage Sal 1 | non traduites | traduction | jusqu'à |
| | | | l'aminoacide 149 |
| Amorce (3') | | | |
| 5' GCGCGTCGACTTACATCGAGGCCTGGCCCAGGCGCAG 3' | | | |
| Site | Fin de D1 | | |
| d'accrochage Sal I | | | |
| Amorce (5') | | | LAG-3 D1D2 |
| identique à LAG-3 D1 | | | |
| | | | depuis la |
| Amorce (3') | | | séquence leader |
| 5' GCGCGTCGACTTAACCCAGAACAGTGAGGTTATAC 3' | | | jusqu'à |
| | | | l'aminoacide 239 |
| Site | Fin de D2 | | |
| d'accrochage Sal 1 | | | |
| Amorce (5') | | | LAG-3 D1-D4 |
| identique à LAG-3 D1 | | | |
| | | | depuis la |
| Amorce (3') | | | séquence leader |
| 5' GCGCGTCGACTTAACCTGGGCTAGACAGCTCTGTG 3' | | | jusqu'à |
| | | | l'aminoacide 412 |
| Site | Fin de D4 | | |
| d'accrochage Sal I | | | |

Les fragments amplifiés résultants ont été digéré par Sal I et insérés dans le site Sal I de pUC 18 (Stratagène).

Les séquences amplifiées ont été vérifiées et les inserts sous-clonés dans le vecteur d'expression pCLH3 AXS V2 DHFR hα IVS comme décrit par COLE et al. (Biotechnology 11, 1014-1024, 1993) (Figure 4).

Ce vecteur est un vecteur d'expression eucaryote multifonctionnel pour l'expression d'ADNc et son amplification dans des cellules eucaryotes. Il possède les caractéristiques suivantes : (i) le promoteur murin du gène de la métallothionéine-1 et une séquence de polyadénylation de SV40 (comprenant un site d'épissage-donneur-accepteur) pour conduire la transcription du gène d'intérêt, (ii) une séquence A d'intervention humaine contenant le site d'épissage-donneur-accepteur du gène de la sous-unité de la glycoprotéine α pour l'obtention de taux élevés de transcription de l'ADNc, (iii) la séquence pML contenant l'origine de replication de pBR 322 et un gène de résistance à l'ampicilline pour l'amplification bactérienne et (iv) une unité de transcription de la dhfr de SV 40 pour conduire la transcription des séquences utilisées pour la sélection et l'amplification des transfectants.

### Expression stable dans les cellules CHO

Les vecteurs d'expression codant pour LAG-3 D1, LAG-3 D1D2 et LAG-3 D1-D4 ont été utilisés pour transfecter des cellules CHO DUKX et ces cellules ont été cultivées sur un milieu sélectif. Les cellules capables de se multiplier dans ces conditions ont été rassemblées et cultivées sur un milieu contenant des quantités croissantes de MTX. Les taux d'expression ont été mesurés par analyse de Western-Blot à l'aide de l'anticorps monoclonal 17B4. Les clones produisant des taux élevés de molécules solubles recombinants dérivées de LAG-3 ont été propagés dans des bioréacteurs et le matériau dérivant de LAG-3 a été purifié par chromatographie d'échange d'ions et immuno-affinité.

Des analyses de Western-Blot ont révélé dans les surnageants de cellules transfectées avec des vecteurs d'expression codant pour LAG-3 D1, LAG-3 D1D2 et LAG-3 D1-D4 des bandes avec des Mr apparents de 15 à 18 kD, 34-36 kD (doublets / et 55 kD (2 bandes possibles). Les Mr respectifs de ces matériaux immunoréactifs correspondaient aux Mr attendus de LAG-3 D1 Ig (139 aminoacides et un site de N-glycosylation putatif), LAG-3 D1D2 Ig (239 aminoacides comportant 3 sites de glycosylation) et LAG-3 D1-D4 Ig (412 aminoacides comportant 4 sites de glycosylation) glycosylés.

### EXEMPLE 4

### Liaison spécifiques des LAG-3 Ig aux cellules exprimant le CMH de Classe II

La réactivité des anticorps monoclonaux et de LAG-3 D1-D4 Ig a été étudiée par immunofluorescence indirecte. Des cellules cibles (4 x 10⁵) ont été mises à incuber pendant 30 minutes à 4°C en présence de LAG-3 D1-D4 Ig, CD8 Ig, un anticorps monoclonal murin, (949) anti-CMH de Classe II humain (DR, DP, DQ) conjugué à du FITC (fluorure d'isothiocyanate) d'un clone Coulter ou de Ig-FITC murin : une immunoglobuline G non pertinente conjuguée à du FITC. Les cellules ont été lavées et incubées à 4°C pendant 30 minutes avec soit un F(ab')₂ polyclonal de chèvre anti-Ig humain conjugué à de la fluorescéine ou un anticoprs polyclonal de chèvre anti-Ig de souris conjugué à de la fluorescéine (clone Coulter).

Pour confirmer la liaison LAG-3 / CMH Classe II, les LAG-3 D1-D4 Ig ont été incubés avec des cellules CMH Classe II positives ou négatives. Quatre lignées de lymphocytes B exprimant le CMH de Classe II(L31, Phil EBV, Raji, Sanchez et Personnaz) ont été traitées avec l'anticorps monoclonal 949 anti-Classe II, ou les surnageants de cellules Cos transfectées avec l'ADN codant soit pour LAG-3 D1-D4 Ig, soit pour CD8 Ig. Les cinq lignées cellules exprimant les différents haplotypes des molécules du CMH de Classe II ont été reconnues par LAG-3 Ig de la même manière que par les anticorps monoclonaux anti-Classe II (témoin positif), tandis que le surnageant contenant CD8 Ig (témoin négatif) ne s'est pas lié à ces lignées cellulaires, comme on pouvait s'y attendre. Quatre lignées cellulaires CMH Classe II négatives (CEM, RJ, HSB2, K562) ont été traitées avec les mêmes réactifs que ci-dessus. Aucune n'a réagi, ni avec les anti-CMH Classe II (témoin négatif), ni avec LAG-3 D1-D4 Ig, ce qui montre que la liaison de LAG-3 D1-D4 est spécifique aux molécules de CMH de Classe II.

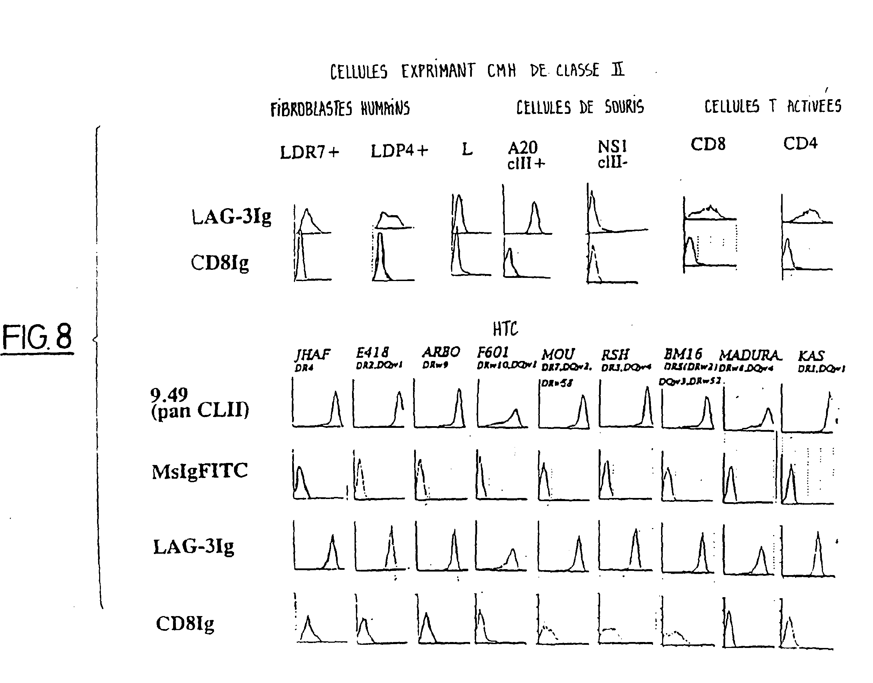

Des expériences supplémentaires ont été réalisées à l'aide de (i) fibroblastes de souris transfectées ou non avec des gènes codant pour DR7 humain ou DP4 humain, (ii) des cellules de souris exprimant ou non les molécules du CMH de Classe II, (iii) des cellules humaines CD4⁺ ou CD8⁺ activées et (iv) des lignées de lymphocytes T exprimant les différents haplotypes des molécules de CMH de Classe II (Figure 8).

Au contraire de CD8 Ig, LAG-3 D1-D4 Ig se lie à toutes les cellules exprimant le CMH de Classe II aussi efficacement que l'anticorps monoclonal 949 anti-CMH Classe II. LAG-3 D1-D4 Ig se lie à tous les haplotypes DR et DP testés, aux molécules du CMH de Classe II humain exprimées par des cellules de souris transfectées, aux molécules du CMH de Classe II murin ainsi qu'aux molécules du CMH de Classe II exprimées par des lymphocytes T CD4⁺ ou CD8⁺.

Ces résultats représentent pour la première fois la preuve que des molécules solubles dérivées d'un ligand du CMH de Classe II sont capables de se fixer aux cellules exprimant le CMH de Classe II.

Des expériences similaires ont montré que LAG-3 D1D2 se liait aux cellules exprimant le CMH Classe II de manière aussi spécifique et avec la même efficacité que LAG-3 D1-D4.

### Activité de liaison de LAG-3Ig et distribution cellulaire des ligands de LAG-3Ig

La capacité de cette immunodhésine à se lier à des ligands cellulaires est mesurée à l'aide d'un sérum de chèvre dirigé contre les immunoglobulines humaines, marqué à la fluorescéine.

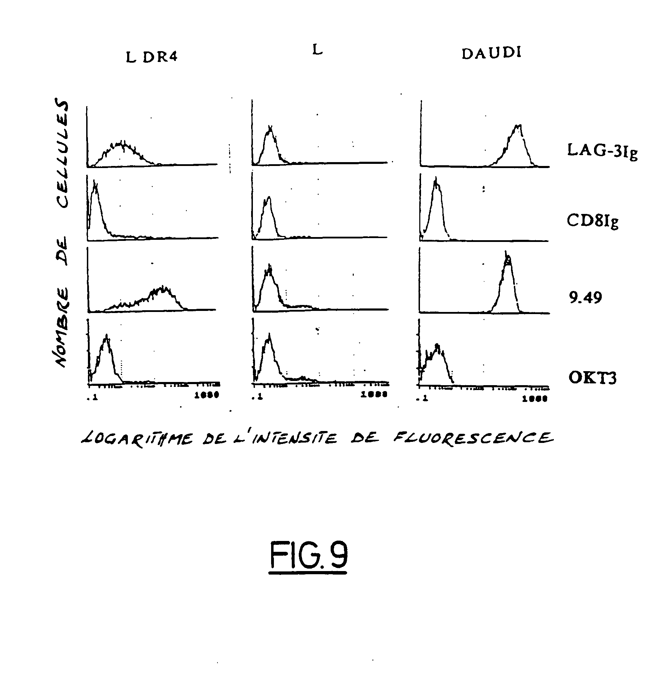

Dans ces expériences, les cellules cibles sont d'abord incubées avec un anticorps monoclonal humain ou une immunoadhésine pendant 30 mn à 4°C dans du RPMI 1640 contenant 10 % de FCS (sérum de veau foetal). Les cellules sont ensuite incubées avec un sérum de chèvre anti-immunoglobulines de souris marqué au FITC (Coulter) pour les anticorps monoclonaux murins ou avec un sérum de chèvre anti-immunoglobulines humaines marqué au FITC (Tago) pour les immunoadhésines. La fluorescence est mesurée après deux lavages en analysant 3 000 cellules sur un cytomètre Elite (Coultronics, Hialeah, FL). La figure 9 indique les taux de fixation de LAG-3Ig, CD8Ig, l'anticorps 949 ou l'anticorps OKT3 (anti-CD3, ATCC), représentés par le nombre de cellules comptées en fonction du logarithme de l'intensité de fluorescence mesurée.

LAG-3Ig se fixe à des fibroblastes murins transfectés pour le gène de la molécule HLA DR₄, et ne se fixe pas sur des cellules non transfectées. CD8Ig est incapable de se lier à des fibroblastes HLA DR₄⁺ dans les mêmes conditions.

La distribution cellulaire des ligands de LAG-3Ig a été évaluée sur un échantillon de populations cellulaires par immunofluorescence.

LAG-3Ig est révélée sur toutes les cellules classe II positives testées, y compris des lignées de cellules B transformées par le virus d'Epstein-Barr (dérivées de donneurs génétiquement non apparentés, incluant 10 lignées homozygotes de typage de DR₁ à DR₁₀), ainsi que sur des cellules T et NK activées.

La figure 9 montre à titre d'exemple la liaison de LAG-3Ig surcellules DAUDI positives pour les antigènes de classe II.

L'intensité moyenne de fluorescence avec LAG-3Ig est similaire à celle observée avec l'anticorps 949 spécifique des antigènes de classe II. La fixation de LAG-3Ig à DR₄ (figure 9), DR₂, DR₇ ou DPw4 (non représenté) exprimés à la surface des fibroblastes murins est au contraire plus faible que celle observée pour l'anticorps 949.

Aucune liaison n'est détectée sur des lignées cellulaires négatives pour les antigènes de classe II d'origine T (cellules T du sang périphérique, lignées CEM, HSB2, REX), d'origine B (lignée RJ 2.2.5), ou d'origine non lymphoïde (lignées humaines K562 d'origine crythro-mycloïde et lignée provenant de cellules de mélanome -(non représentées)).

Par ailleurs, LAG-3Ig se fixe à des molécules classe II du CMH xénogéniques, tels que les antigènes exprimés par le lymphome murin A 20 et les classes II de singe exprimés par des blastes stimulés à la phytohémaglutinine (données non représentées).

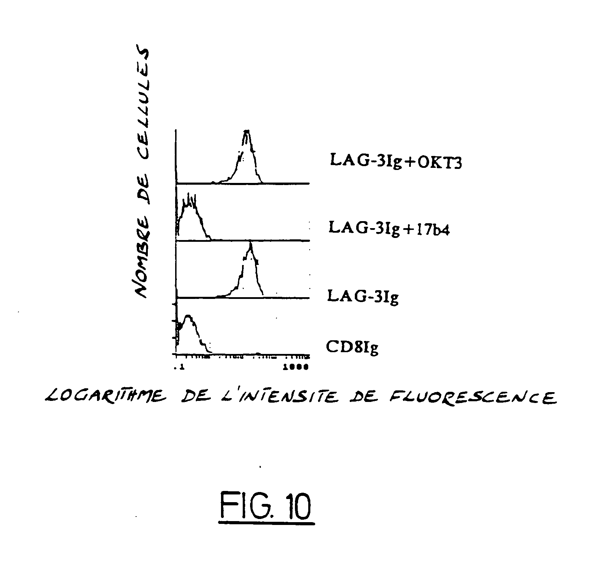

La spécificité de fixation de LAG-3Ig a été également vérifiée en utilisant l'anticorps monoclonal 17B4, dont la capacité à bloquer les interactions LAG-3/CMH classe II dans des tests d'adhésion cellulaire a été auparavant démontrée (figure 10).

Dans ces expériences, les molécules LAG-3Ig sont préincubées pendant 30 minutes à 4°C soit avec du milieu seul, soit avec 17B4 (1 mg/ml) soit avec OKT3 (1 mg/ml) avant d'être mises en présence des cellules DAUDI.

La figure 10 montre qu'une préincubation de LAG-3Ig avec 17B4 inhibe la fixation aux cellules classe II⁺, alors qu'aucune inhibition n'est détectée avec le contrôle OKT3.

### EXEMPLE 5

### Inhibition de l'interaction LAG-3/CMH classe II par des fragments solubles de LAG-3.

L'inhibition de l'interaction LAG-3/CMH classe II par les fragments solubles de LAG-3 peut s'observer directement au niveau de la fixation de LAG-3 Ig sur les CMH classe II, par des expériences de compétition avec les fragments solubles.

Pour vérifier si les fragments solubles LAG-3D₁D₂ produits par les CHO pouvaient déplacer la liaison d'immunoadhésines dérivées de LAG-3, les essais suivants ont été réalisés :
Des cellules DAUDI sont incubées avec des fragments LAG3-D₁D₂ solubles, de façon à permettre la fixation de ces molécules aux antigènes de classe II du CMH exprimés à la surface des cellules DAUDI.
Dans une seconde étape, les cellules sont incubées en présence de LAG-3D₁D₄Ig sous forme dimérique ou de LAG-3D₁D₂Ig sous forme monomérique.

La fixation de ces immunoadhésines dérivées de LAG-3 est mesurée à l'aide d'un F(ab')₂ de chèvre anti Ig humain conjugué à de la fluorescéine (GAH FITC).

Les groupes contrôles sont représentés par des cellules DAUDI incubées avec du LAG-3D₁D₄Ig dimérique ou du LAG-3 D₁D₂Ig monomérique sans préincubation avec les fragments LAG-3D₁D₂ solubles.

Les résultats sont reportés dans le tableau 5 qui indique que les fragments LAG-3D₁D₂ solubles sont capables de déplacer les immunoadhésines dérivées de LAG-3 sous forme mono- ou dimérique.

**TABLEAU 5**

| **Réactifs** | **Détection** | **Moyenne de fluorescence** | **Conclusion** |
|---|---|---|---|
| --- | GAH-FITC | 0.3 | GAH n'interfère pas |
| LAG-3D1D4Ig dimérique | GAH-FITC | 20.8 | La liaison de CHO/LAG-3D1D2 inhibe la liaison de LAG-3D1D4Ig dimérique (58 %) (58 %) |
| CHO/LAG-3D1D2, puis LAG-3D1D4Ig dimérique | GAH-FITC | 8.5 | |
| LAG-3D1D2Ig monomérique | GAH-FITC | 62.5 | La liaison de CHO/LAG-3D1D2 inhibe la liaison de LAG-3D1D2Ig monomérique (27 %) |
| CHO/LAG-3D1D2, puis LAG-3D1D2Ig monomérique | GAH-FITC | 10.9 | |

Ces données confirment que les fragments LAG-3D₁D₂ solubles se fixent sur les molécules classe II du CMH.

### Inhibition de l'interaction LAG-3 / CMH Classe II et CD4 / CMH Classe II

La formation de rosettes entre des cellules Cos transfectées ar LAG-3 de type sauvage et des lymphocytes B transformés par l'EBV exprimant les molécules du CMH Classe II a été démontré par BAIXERAS et al. (2). Cette interaction est inhibée à la fois par des anticorps monoclonaux anti-LAG-3 et anti-MCH Classe II.

La méthode décrite dans cette publication a été modifiée en remplaçant la visualisation et le comptage des cellules Cos se liant aux lymphocytes B par le comptage de la radio-activité restante après incubation de lymphocytes B marqués au ⁵¹Cr avec des cellules Cos exprimant LAG-3 (essai de liaison).

Les éventuels effets inhibiteurs des molécules solubles dérivées de LAG-3 sur l'interaction LAG-3 / CMH Classe II, mais aussi sur l'interaction CD4 / CMH Classe II ont été étudiés.

Des cellules Cos ont été transfectées avec un vecteur d'expression approprié (codant pour LAG-3 type sauvage ou CD4). Deux jours plus tard, les cellules Cos ont été traitées à la trypsine et étalées à nouveau à raison de 0,05 x 10⁶ cellules / puits sur des plaques pour culture de tissus à fond plat à 12 puits. 24 heures plus tard, des cellules Daudi marquées au ⁵¹Cr (5,5 x 10⁶) ont été incubées sur cette monocouche de cellules Cos (vol. final : 1 ml) pendant 1 heure. Les cellules B cibles ont alors été aspirées et les puits lavés à 5 à 7 reprises, en y ajoutant doucement goutte à goutte 1 ml de milieu. Les bords des puits ont été lavés par aspiration à l'aide d'une pipette Pasteur. Les cellules restantes ont été lysées avec 1 ml de PBS ; 1% Triton pendant 15 minutes à 37°C. Les lysats ont été centrifugés à 3000 tpm pendant 10 minutes et 100 µl du surnageant résultant ont été comptés.

LAG-3 D1-D4 Ig a été utilisé pour inhiber l'interaction LAG-3 / CMH Classe II et CD4 / CMH Classe II dans l'essai de liaison au ⁵¹Cr. Les CD8 Ig et IgG1 humaines ont été testées en parallèle et utilisées comme témoins négatifs.

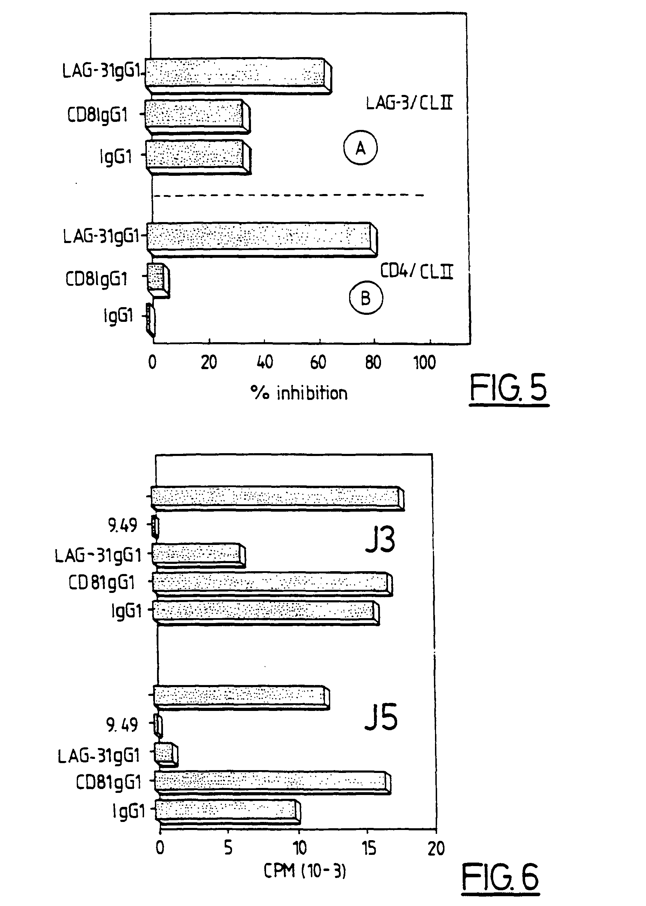

Une inhibition significative de l'interaction LAG-3 / Classe II par LAG-3 D1-D4 Ig a été détectée (Fig. 5A). Cependant, l'interaction LAG-3 / CMH Classe II peut être inhibée partiellement et de manière non spécifique par CD8 Ig et les IgG1 humaines. D'autre part, LAG-3 Ig s'est avéré un inhibiteur potentiel de l'interaction CD4 / Classe II (Figure 5B) dans des conditions expérimentales où l'interaction CD4 / CMH Classe II n'était pas modifiée par CD8 Ig ou des IgGl humaines. Ceci suggère que l'interaction LAG-3 / Classe II est plus faible que l'interaction CD4 / Classe II. Ces résultats représentent la première preuve d'une compétition possible de molécules soluble sur une interaction du CMH de Classe II avec ses ligands.

### EXEMPLE 6

### Activité immuno-suppressive de LAG-3 D1-D4 Ig

Des essais fonctionnels ont été effectués à l'aide des essais de prolifération décrits ci-dessus pour l'activité biologique des anticorps monoclonaux anti-LAG-3.

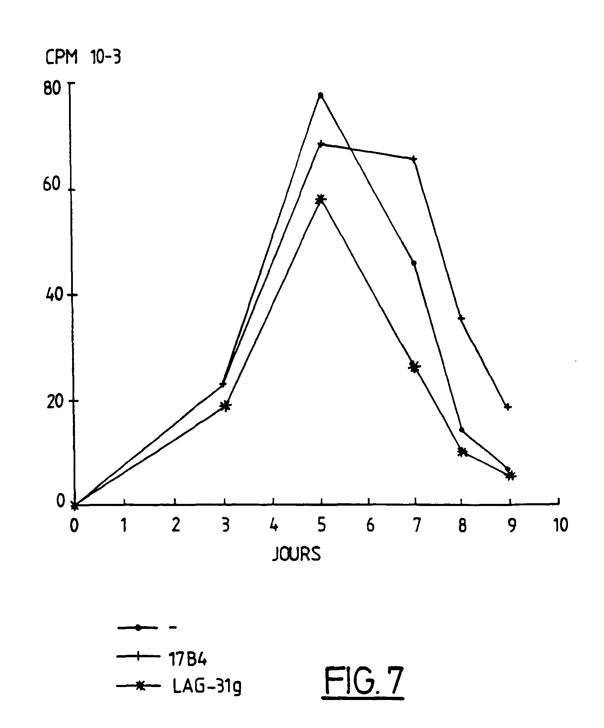

3 jours et 5 jours (J3 et J5) après la stimulation antigénique, LAG-3 D1-D4 Ig a montré une inhibition forte de la prolifération du clone 28 tandis que CD8 Ig et IgG humaines n'avaient aucun effet (Figure 6). Des expériences similaires ont été réalisées avec le clone 154 (Figure 7) et ont montré une inhibition partielle en présence de LAG-3 Ig. Un contrôle réalisé avec les anticorps monoclonaux anti-LAG-3 avait les effets inverses, comme observé précédemment.

Une inhibition significative de la prolifération cellulaire de cellules incubées en présence de LAG-3 D1-D4 Ig a également été observée pour le clone 28.

Ces observations montrent que LAG-3 D1-D4 Ig est un immuno-suppresseur potentiel de la prolifération de lymphocytes T stimulés par un antigène, et indiquent que LAG-3 pourrait agir comme un "extincteur" de la réponse immunitaire secondaire induite par les lymphocytes T auxilliaires CD4⁺ activés.

### Rôle de LAG-3Ig dans la régulation négative des réponses immunitaires des cellules T.

Pour démontrer qu'une forme soluble de LAG-3, mimant les fonctions de la molécule membranaire pouvait inhiber l'activation de clones T CD₄+ stimulés par un antigène, les essais suivants ont été réalisés sur le clone T 154 : les cellules T sont préalablement incubées avec une quantité saturante de LAG-3Ig (100 nM). Les cellules sont ensuite lavées deux fois avec du RPMI froid et incubées avec 10 µg/ml d'anticorps de chèvre dirigés contre les immunoglobulines humaines (Tago) à 4°C, pendant 30 minutes.
Après deux nouveaux lavages, les cellules sont resuspendues dans du RPMI contenant 10 % de sérum de veau foetal et incubée 2 heures à 37°C avant l'addition du signal. Pour coupler ("cross-link") les anticorps monoclonaux, on utilise un anticorps de chèvre anti-souris à 10 µg/ml (Tago).

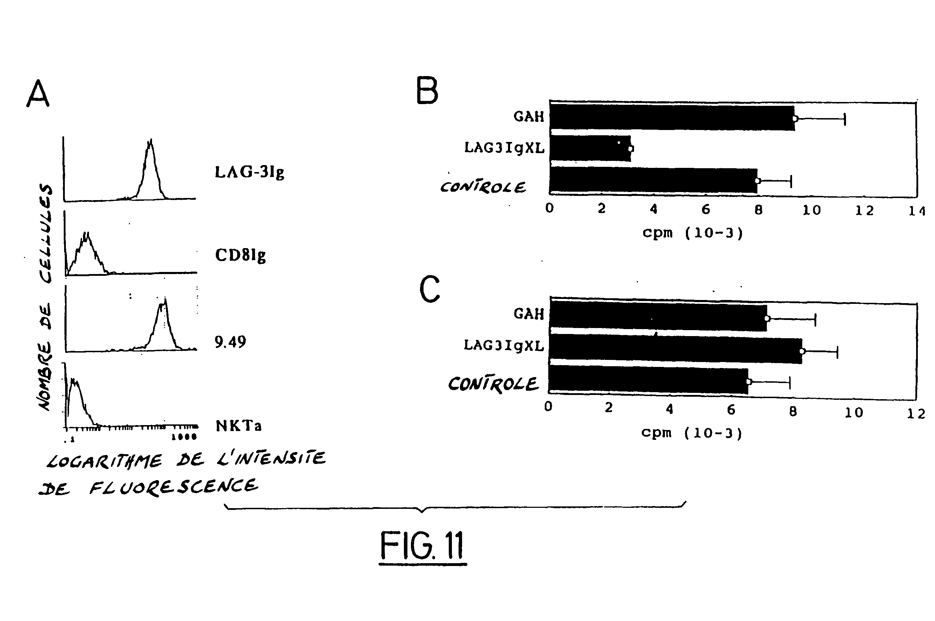

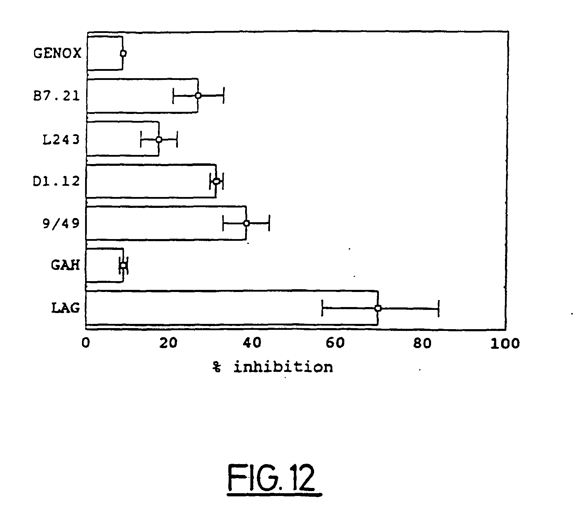

La figure 11 représente une expérience dans laquelle le clone T154 a été préincubé avec LAG-3Ig lié ("cross-linked") à un second réactif (sérum polyclonal spécifique pour la région constante des immunoglobulines humaines). Le taux de fixation de LAG-3Ig aux cellules est mesuré par immunofluorescence (figure 11A). La figure 11B montre qu'une inhibition de plus de 50 % de la prolifération du clone T154 est produite par LAG-3Ig. Dans les mêmes conditions expérimentales, aucun effet n'est observé avec le contrôle CD8Ig ou avec LAG-3Ig sans "cross-linking" (non représenté sur la figure).

La figure 11 C montre également qu'aucun effet n'est observé lorsque LAG-3Ig est utilisé pour lier ("cross-link") les molécules de classe II du CMH exprimées par des cellules B présentatrices d'antigène.

Les effets éventuels d'anticorps monoclonaux anti-classe II liés ("cross-linked") au niveau de la prolifération des cellules T ont été comparés à ceux de LAG-3Ig. Une faible inhibition (inférieure à 50 %) est observée avec l'anticorps 949 et l'anticorps D1.12 (anti-DR) liés à un sérum polyclonal de chèvre anti-souris (figure 12). L'inhibition de la prolifération est donc épitope dépendante, l'effet le plus important étant obtenu avec l'épitope de LAG-3 spécifique de la liaison aux classes II.

Les effets de LAG-3Ig sur la prolifération des cellules T ont également été étudiés en utilisant différents signaux sur un autre clone T CD₄⁺, le clone TDEL spécifique du peptide 34-53 de la protéine de myéline basique.

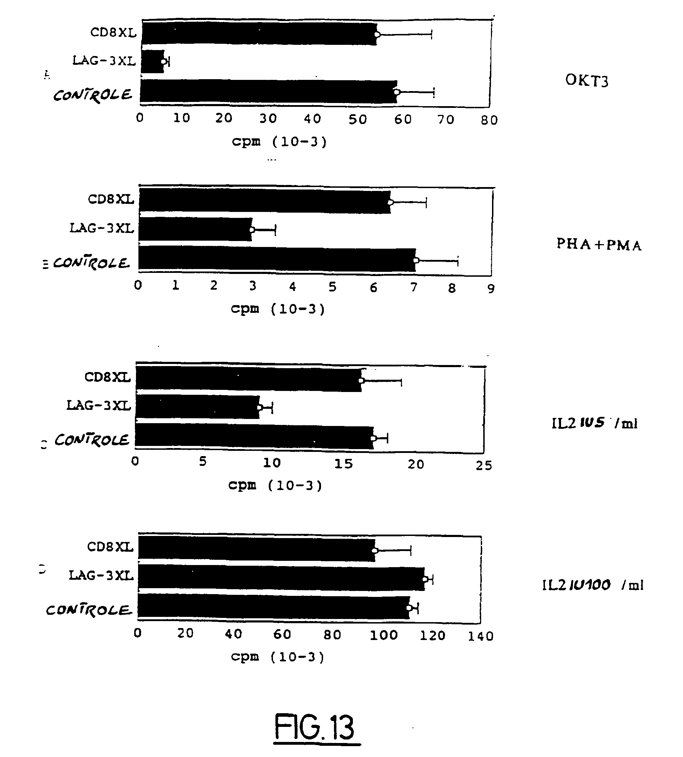

Une inhibition de la prolifération est observée (n = 2) quand TDEL est stimulé avec l'antigène (non représenté), avec l'OKT3 immobilisé (figure 13 A), avec des lectines (PHA + PMA) (figure 13 B) et avec 5 UI/ml d'IL₂ (figure 13 C). Aucune inhibition n'est observée avec 100 UI/ml d'IL₂ (figure 13 D).

En conclusion, l'ensemble de ces résultats indiquent que LAG-3 et les molécules de CMH classe II, qui sont chacun des antigènes d'activation des cellules T, sont assimilables à des molécules d'effecteurs impliqués dans la phase d'inactivation des réponses des cellules T. Par ailleurs, ces résultats illustrent l'importance des interactions entre cellules T dans le contrôle de la réponse immunitaire cellulaire.

### EXEMPLE 7

### Stimulation de la cytotoxicité cellulaire par LAG-3Ig

Le rôle de LAG-3Ig au niveau de la cytotoxicité cellulaire est étudié sur deux types de cellules effectrices :
- des lymphocytes du sang périphérique humains (PBL) fraîchement prélevés,
- des cellules de la lignée S1B5, (clone de cellules NK humaines).

L'activité cytotoxique de ces cellules est mesurée par comptage du ⁵¹Cr relargué dans le milieu par des cellules cibles préalablement marquées, en présence ou en absence de LAG-3Ig dans le milieu.

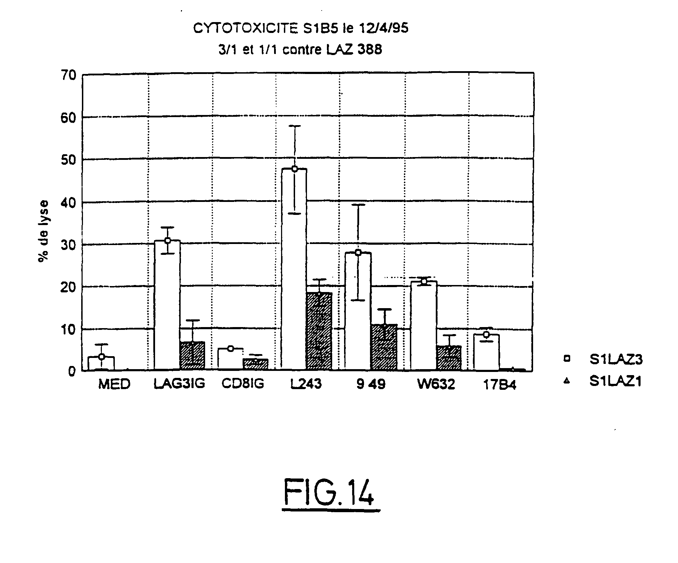

La figure 14 montre le taux de cytotoxicité de S1B5 pour une lignée de cellules B humaines transformées par le virus d' Epstein-Barr et portant les antigènes de classe I et II du complexe majeur d'histocompatibilité (lignée LAZ 388), en fonction de différents réactifs ajoutés aux cultures.

Les mesures sont réalisées après 4 heures de co-culture pour des rapports cellules effectrices/cellules cibles (S1B5/LAZ 388) de 3/1 (colonnes blanches) ou de 1/1 (colonnes hachurées).

Les contrôles négatifs sont constitués par du milieu seul (MED), l'immunoadhésine CD8Ig, et l'anticorps monoclonal 17.B4 (anti-LAG-3).

Les contrôles positifs sont constitués par trois anticorps monoclonaux différents :
- l'anticorps L243 dirigé contre les antigènes de classe II DR,
- l'anticorps 9.49 dirigé contre les antigènes de classe II DR, DP, DQ,
- l'anticorps W632 dirigé contre les antigènes de classe I du complexe majeur d'histocompatibilité humain.

Des anticorps anti-HLA classe I (W632) ou classe II (L243) augmentent la lyse des cellules cibles (et pas le contrôle 17B4). L'immunoadhésine LAG-3Ig augmente la lyse ; le contrôle CD8Ig n'a pas d'effet.

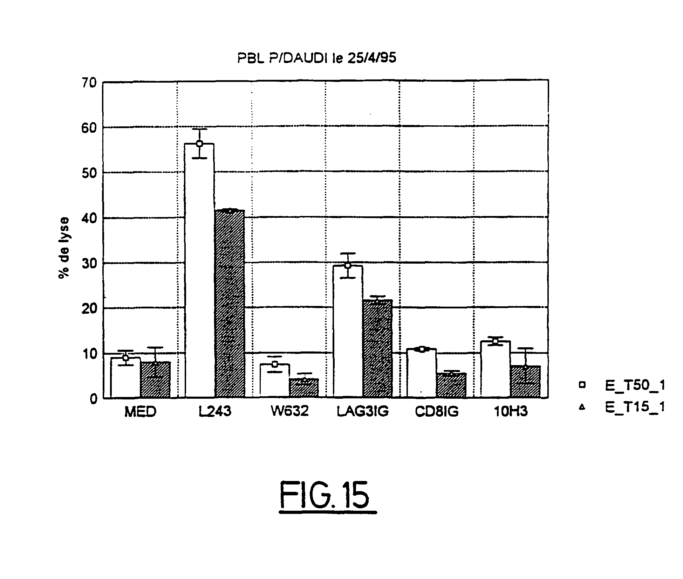

La figure 15 indique les résultats d'une expérience analogue à la précédente dans laquelle est mesurée la cytotoxicité des PBL envers des cellules DAUDI (HLA Classe I⁻), pour des rapports effecteurs/cibles de 50/1 (colonnes blanches) et de 15/1 (colonnes hachurées). Les réactifs ajoutés dans le milieu sont les mêmes que ceux utilisés dans la première expérience, excepté l'anticorps 9.49 et l'anticorps 17.B4. L'anticorps 10H3 est une immunoglobuline d'isotype IgG1 spécifique de l'antigène de surface CD45. Il est utilisé comme contrôle négatif.

Aucun changement n'est observé avec un anticorps dirigé contre les antigènes de classe I du complexe majeur d'histocompatibilité (W632).

Les données de ces deux séries de mesures indiquent que, comparé aux contrôles négatifs, LAG-3Ig active la cytotoxicité des cellules NK. Cet effet est similaire à celui observé avec des anticorps dirigés contre les molécules de classe II du CMH.

### LISTE DES SEQUENCES

### I - INFORMATIONS GENERALES

(1) DEMANDEURS : INSTITUT GUSTAVE ROUSSY / INSERM, APPLIED RESEARCH SYSTEMS ARS HOLDING N.V.
(2) TITRE DE L'INVENTION :
   Fractions polypeptidiques solubles dérivées de LAG-3 ; composition thérapeutique, utilisation des anticorps anti-LAG-3.
(3) NOMBRE DE SEQUENCES : 1

### II - INFORMATIONS POUR LA SEQUENCE SEQ ID N° 1

### CARACTERISTIQUES DE LA SEQUENCE

Type : Nucléotide
Longueur : 476
Nombre de brins : double
Configuration : linéaire
Type de molécule : ADNc
Organisme : homo sapiens
Tissu : lymphocytes T
Nom : LAG-3
Description de la séquence :

Leader peptide
-22 Met-Trp-Glu-Ala-Gln-Phe-Leu-Gly-Leu-Leu-Phe-Leu-Gln-Pro-Leu-
Trp-Val-Ala-Pro-Val-Lys-Pro
D1
1 Leu-Gln-Pro-Gly-Ala-Glu-Val-Pro-Val-Val-Trp-Ala-Gln-Glu-Gly-
16 Ala-Pro-Ala-Gln-Leu-Pro-Cys-Ser-Pro-Thr-Ile-Pro-Leu-Gln-Asp-
31 Leu-Ser-Leu-Leu-Arg-Arg-Ala-Gly-Val-Thr-Trp-Gln-His-Gln-Pro-
46 Asp-Ser-Gly-Pro-Pro-Ala-Ala-Ala-Pro-Gly-His-Pro-Leu-Ala-Pro-
61 Gly-Pro-His-Pro-Ala-Ala-Pro-Ser-Ser-Trp-Gly-Pro-Arg-Pro-Arg-

76 Arg-Tyr-Thr-Val-Leu-Ser-Val-Gly-Pro-Gly-Gly-Leu-Arg-Ser-Gly-

91 Arg-Leu-[?][?]o-Leu-Gln-Pro-Arg-Val-Gln-Leu-Asp-Glu-Arg-Gly-Arg-

106 Gln-Arg-Gly-Asp-Phe-Ser-Leu-Trp-Leu-Arg-Pro-Ala-Arg-Arg-Ala-

121 Asp-Ala-Gly-Glu-Tyr-Arg-Ala-Ala-Val-His-Leu-Arg-Asp-Arg-Ala-

136 Leu-Ser-Cys-Arg-Leu-Arg-Leu-Arg-Leu-Gly-Gln-Ala-Ser-Met 149
------------------------------------------------------------
D2
150 Thr-

151 Ala-Ser-Pro-Pro-Gly-Ser-Leu-Arg-Ala-Ser-Asp-Trp-Val-Ile-Leu-

166 Asn-Cys-Ser-Phe-Ser-Arg-Pro-Asp-Arg-Pro-Ala-Ser-Val-His-Trp-

181 Phe-Arg-Asn-Arg-Gly-Gln-Gly-Arg-Val-Pro-Val-Arg-Glu-Ser-Pro-

196 His-His-His-Leu-Ala-Glu-Ser-Phe-Leu-Phe-Leu-Pro-Gln-Val-Ser-

211 Pro-Met-Asp-Ser-Gly-Pro-Trp-Gly-Cys-Ile-Leu-Thr-Tyr-Arg-Asp-

226 Gly-Phe-Asn-Val-Ser-Ile-Met-Tyr-Asn-Leu-Thr-Val-Leu-Gly 239
------------------------------------------------------------
D3
240 Leu-

241 Glu-Pro-Pro-Thr-Pro-Leu-Thr-Val-Tyr-Ala-Gly-Ala-Gly-Ser-Arg-

256 Val-Gly-Leu-Pro-Cys-Arg-Leu-Pro-Ala-Gly-Val-Gly-Thr-Arg-Ser-

271 Phe-Leu-Thr-Ala-Lys-Trp-Thr-Pro-Pro-Gly-Gly-Gly-Pro-Asp-Leu-

286 Leu-Val-Thr-Gly-Asp-Asn-Gly-Asp-Phe-Thr-Leu-Arg-Leu-Glu-Asp-

301 Val-Ser-Gln-Ala-Gln-Ala-Gly-Thr-Tyr-Thr-Cys-His-Ile-His-Leu-

316 Gln-Glu-Gln-Gln-Leu-Asn-Ala-Thr-Val-Thr-Leu-Ala-Ile-Ile-Thr 330
------------------------------------------------------------

D4
331 Val-Thr-Pro-Lys-Ser-Phe-Gly-Ser-Pro-Gly-Ser-Leu-Gly-Lys-Leu-

346 Leu-Cys-Glu-Val-Thr-Pro-Val-Ser-Gly-Gln-Glu-Arg-Phe-Val-Trp-

361 Ser-Ser-Leu-Asp-Thr-Pro-Ser-Gln-Arg-Ser-Phe-Ser-Gly-Pro-Trp-

376 Leu-Glu-Ala-Gln-Glu-Ala-Gln-Leu-Leu-Ser-Gln-Pro-Trp-Gln-Cys-

391 Gln-Leu-Tyr-Gln-Gly-Glu-Arg-Leu-Leu-Gly-Ala-Ala-Val-Tyr-Phe-

406 Thr-Glu-Leu-Ser-Ser-Pro-Gly 412
------------------------------------------------------------------------
Transmembrane
413 Ala-Gln-Arg-Ser-Gly-Arg-Ala-Pro-

421 Gly-Ala-Leu-Pro-Ala-Gly-His-Leu-Leu-Leu-Phe-Leu-Thr-Leu-Gly-

436 Val-Leu-Ser-Leu-Leu-Leu-Leu-Val-Thr-Gly-Ala-Phe-Gly-Phe-His-

451 Leu-Trp 452
------------------------------------------------------------------------
Intracytoplasmic
453 Arg-Arg-Gln-Trp-Arg-Pro-Arg-Arg-Phe-Ser-Ala-Leu-Glu-

466 Gln-Gly-Ile-His-Pro-Arg-Arg-Leu-Arg-Ala-Arg 476

### REFERENCES BIBLIOGRAPHIQUES

1. TRIEBEL T. et al., 1990, J. Exp. Med. 171, 1393-1405
2. BAIXERAS E. et al., 1992, J. Exp. Med. 176, 327-337
3. COSGROVE D. et al., 1991, Cell 66, 1051-1066
4. RAHEMTULLA A. et al., 1991, Nature 353, 180-184
5. TRAUNECKER A. et al., 1988, Nature 331, 84-86
6. BENEDICT A.A. et al., 1967, Methods in Immunology 1, 197-306 (1967)
7. YELTON D.E. et al., Ann. Rev. of Biochem. 50, 657-680 (1981)
8. HUARD B. et al., Immunogenetics 39:213
9. MANIATIS T. et al. (1982), Molecular cloning : A laboratory manual - Cold Spring Harbor Laboratory, New-York.
10. SEED B., 1987, Nature 329, 840-842
11. COLE S.C. et al., Biotechnology 11, 1014-1024, 1993
12. COLE S.C. et al., Biotechnology 11, 1014-1024, 1993.

**TABLEAU N° 1**

| Nom de l'atome | x | y | z | nom et n° du résidu | | type, charge et n° de l'atome | | |
|---|---|---|---|---|---|---|---|---|
| N | 25.172370911 | 27.259836197 | 67.855064392 | AP-n | 40 | n3 | -0.5000 | 1 |
| HN2 | 25.667764664 | 26.471963882 | 67.420585632 | AP-n | 40 | hn | 0.1300 | 2 |
| CA | 24.625223160 | 26.867494583 | 69.180244446 | AP-n | 40 | ca | 0.1200 | 3 |
| HN1 | 24.393711090 | 27.474891663 | 67.220008850 | AP-n | 40 | hn | 0.1300 | 4 |
| HA | 23.936895370 | 27.680395126 | 69.464080811 | AP-n | 40 | h | 0.0700 | 5 |
| C | 25.662780762 | 26.773513794 | 70.350120544 | AP-n | 40 | c' | 0.3800 | 6 |
| O | 25.295415878 | 27.090747833 | 71.482070923 | AP-n | 40 | o' | -0.4100 | 7 |
| CB | 23.766021729 | 25.587018967 | 68.990669250 | AP-n | 40 | c2 | -0.2600 | 8 |
| HB1 | 23.060285568 | 25.727443695 | 68.152351379 | AP-n | 40 | h | 0.0700 | 9 |
| HB2 | 24.413969040 | 24.744903564 | 68.686981201 | AP-n | 40 | h | 0.0700 | 10 |
| CG | 22.921775818 | 25.153419495 | 70.196960449 | AP-n | 40 | c- | 0.3400 | 11 |
| OD1 | 22.069602966 | 25.929233551 | 70.676017761 | AP-n | 40 | o- | -0.5700 | 12 |
| OD2 | 23.115716934 | 24.009321213 | 70.667663574 | AP-n | 40 | o- | -0.5700 | 13 |
| N | 26.906179428 | 26.304588318 | 70.124969482 | SER | 41 | n | -0.5000 | 14 |
| CA | 27.860145569 | 25.912786484 | 71.207519531 | SER | 41 | ca | 0.1200 | 15 |
| HN | 27.120641708 | 26.221813202 | 69.126319885 | SER | 41 | hn | 0.2800 | 16 |
| HA | 27.374551773 | 25.088045120 | 71.766326904 | SER | 41 | h | 0.1000 | 17 |
| C | 28.252065659 | 27.005065918 | 72.271789551 | SER | 41 | c' | 0.3800 | 18 |
| O | 27.987834930 | 28.200620651 | 72.115295410 | SER | 41 | o' | -0.3800 | 19 |
| CB | 29.093601227 | 25.298025131 | 70.494880676 | SER | 41 | c2 | -0.1700 | 20 |
| HB1 | 28.786190033 | 24.599395752 | 69.690521240 | SER | 41 | h | 0.1000 | 21 |
| HB2 | 29.691858292 | 26.092912674 | 70.004081726 | SER | 41 | h | 0.1000 | 22 |
| OG | 29.905221939 | 24.578149796 | 71.424118042 | SER | 41 | oh | -0.3800 | 23 |
| HG | 30.662555695 | 24.231645584 | 70.939277649 | SER | 41 | ho | 0.3500 | 24 |
| N | 28.857526779 | 26.558052063 | 73.387054443 | GLY | 42 | n | -0.5000 | 25 |
| CA | 29.199718475 | 27.440891266 | 74.535232544 | GLY | 42 | cg | 0.0200 | 26 |
| HN | 29.251720428 | 25.616510391 | 73.267890930 | GLY | 42 | hn | 0.2800 | 27 |
| HA1 | 28.520187378 | 28.312047958 | 74.591857910 | GLY | 42 | h | 0.1000 | 28 |
| HA2 | 28.983697891 | 26.890144348 | 75.468444824 | GLY | 42 | h | 0.1000 | 29 |
| C | 30.691051483 | 27.875793457 | 74.601707458 | GLY | 42 | c' | 0.3800 | 30 |
| O | 31.504199982 | 27.026502609 | 74.980445862 | GLY | 42 | o' | -0.3800 | 31 |
| N | 31.113182068 | 29.132183075 | 74.266487122 | PRO | 43 | n | -0.4200 | 32 |
| CA | 32.558349609 | 29.476200104 | 74.126792908 | PRO | 43 | ca | 0.0600 | 33 |
| HA | 33.096603394 | 28.605407715 | 73.708091736 | PRO | 43 | h | 0.1000 | 34 |
| CD | 30.214075089 | 30.174203873 | 73.722633362 | PRO | 43 | c2 | 0.0600 | 35 |
| HD1 | 29.467987061 | 30.516298294 | 74.466743469 | PRO | 43 | h | 0.1000 | 36 |
| HD2 | 29.664882660 | 29.799777985 | 72.838768005 | PRO | 43 | h | 0.1000 | 37 |
| C | 33.318023682 | 29.988374710 | 75.414916992 | PRO | 43 | c' | 0.3800 | 38 |
| O | 32.682361603 | 30.557033539 | 76.312332153 | PRO | 43 | o' | -0.3800 | 39 |
| CB | 32.483139038 | 30.574260712 | 73.043136597 | PRO | 43 | c2 | -0.2000 | 40 |
| HB1 | 33.350620270 | 31.263189316 | 73.049049377 | PRO | 43 | h | 0.1000 | 41 |
| HB2 | 32.463790894 | 30.110534668 | 72.036743164 | PRO | 43 | h | 0.1000 | 42 |
| CG | 31.160949707 | 31.299722672 | 73.307487488 | PRO | 43 | c2 | -0.2000 | 43 |
| HG1 | 31.279897690 | 32.024162292 | 74.137779236 | PRO | 43 | h | 0.1000 | 44 |
| HG2 | 30.794561386 | 31.862808228 | 72.428352356 | PRO | 43 | h | 0.1000 | 45 |
| N | 34.683673859 | 29.902477264 | 75.503486633 | PRO | 44 | n | -0.4200 | 46 |
| CA | 35.485736847 | 30.679145813 | 76.490524292 | PRO | 44 | ca | 0.0600 | 47 |
| HA | 35.018527985 | 30.645456314 | 77.491592407 | PRO | 44 | h | 0.1000 | 48 |
| CD | 35.509281158 | 29.014368057 | 74.655700684 | PRO | 44 | c2 | 0.0600 | 49 |
| HD1 | 35.411357880 | 29.247959137 | 73.577461243 | PRO | 44 | h | 0.1000 | 50 |
| HD2 | 35.214973450 | 27.955932617 | 74.801994324 | PRO | 44 | h | 0.1000 | 51 |
| C | 35.700843811 | 32.172924042 | 76.063224792 | PRO | 44 | c' | 0.3800 | 52 |
| O | 36.448230743 | 32.477428436 | 75.126922607 | PRO | 44 | o' | -0.3800 | 53 |
| CB | 36.779544830 | 29.842718124 | 76.547348022 | PRO | 44 | c2 | -0.2000 | 54 |
| HB1 | 37.662769318 | 30.430650711 | 76.863670349 | PRO | 44 | h | 0.1000 | 55 |
| HB2 | 36.667564392 | 29.027103424 | 71.288825989 | PRO | 44 | h | 0.1000 | 56 |
| CG | 36.940769196 | 29.250995636 | 75.143180847 | PRO | 44 | c2 | -0.2000 | 57 |
| HG1 | 37.446662903 | 29.982709885 | 74.483322144 | PRO | 44 | h | 0.1000 | 58 |
| HG2 | 37.553295135 | 28.329860667 | 75.134750366 | PRO | 44 | h | 0.1000 | 59 |
| N | 35.026676178 | 33.104183197 | 76.753837585 | ALA | 45 | n | -0.5000 | 60 |
| CA | 35.034278870 | 34.544979095 | 76.400695801 | ALA | 45 | ca | 0.1200 | 61 |
| HN | 34.452354431 | 32.747509003 | 77.536170959 | ALA | 45 | hn | 0.2800 | 62 |
| HA | 35.105010986 | 34.659946442 | 75.298950195 | ALA | 45 | h | 0.1000 | 63 |
| C | 36.209384918 | 35.322113037 | 77.076705933 | ALA | 45 | c' | 0.3800 | 64 |
| O | 36.163528442 | 35.637268066 | 78.268325806 | ALA | 45 | o' | -0.3800 | 65 |
| CB | 33.646369934 | 35.083190918 | 76.800727844 | ALA | 45 | c3 | -0.3000 | 66 |
| HB1 | 33.534698486 | 36.150661469 | 76.535202026 | ALA | 45 | h | 0.1000 | 67 |
| HB2 | 32.828392029 | 34.539138794 | 76.290328979 | ALA | 45 | h | 0.1000 | 68 |
| HB3 | 33.465579987 | 35.001335144 | 77.890525818 | ALA | 45 | h | 0.1000 | 69 |
| N | 37.266757965 | 35.613758087 | 76.297294617 | ALA | 46 | n | -0.5000 | 70 |
| HN | 37.216701508 | 35.231555939 | 75.346885681 | ALA | 46 | hn | 0.2800 | 71 |
| CA | 38.489383698 | 36.310386658 | 76.786270142 | ALA | 46 | ca | 0.1200 | 72 |
| HA | 38.262126923 | 36.871456146 | 77.716934204 | ALA | 46 | h | 0.1000 | 73 |
| C | 39.058414459 | 37.311935425 | 75.727844238 | ALA | 46 | c' | 0.3800 | 74 |
| O | 38.922710419 | 37.100418091 | 74.516731262 | ALA | 46 | o' | -0.3800 | 75 |
| CB | 39.526046753 | 35.215301514 | 77.108406067 | ALA | 46 | c3 | -0.3000 | 76 |
| HB1 | 40.446556091 | 35.633434296 | 77.555480957 | ALA | 46 | h | 0.1000 | 77 |
| HB2 | 39.131206512 | 34.469978333 | 77.822120667 | ALA | 46 | h | 0.1000 | 78 |
| HB3 | 39.821903229 | 34.663463593 | 76.197715759 | ALA | 46 | h | 0.1000 | 79 |
| N | 39.737388611 | 30.384365082 | 76.180320740 | ALA | 47 | n | -0.5000 | 80 |
| CA | 40.295833588 | 39.429889679 | 75.275863647 | ALA | 47 | ca | 0.1200 | 81 |
| HN | 39.717037201 | 38.512592316 | 77.196357727 | ALA | 47 | hn | 0.2800 | 82 |
| HA | 39.901103973 | 39.319335938 | 74.245994568 | ALA | 47 | h | 0.1000 | 83 |
| C | 41.869789124 | 39.413467407 | 75.170806885 | ALA | 47 | c' | 0.3800 | 84 |
| O | 42.518333435 | 40.166862488 | 75.906578064 | ALA | 47 | o' | -0.3800 | 85 |
| CB | 39.722030640 | 40.769996643 | 75.786285400 | ALA | 47 | c3 | -0.3000 | 86 |
| HB1 | 40.045078278 | 41.611721039 | 75.145561218 | ALA | 47 | h | 0.1000 | 87 |
| HB2 | 38.615306854 | 40.778987885 | 75.787467957 | ALA | 47 | h | 0.1000 | 88 |
| HB3 | 40.059757233 | 41.007873535 | 76.813346863 | ALA | 47 | h | 0.1000 | 89 |
| N | 42.537422180 | 38.621597290 | 74.274406433 | PRO | 48 | n | -0.4200 | 90 |
| CA | 44.009857178 | 38.718185425 | 74.045745850 | PRO | 48 | ca | 0.0600 | 91 |
| HA | 44.539390564 | 38.855972290 | 75.008773804 | PRO | 48 | h | 0.1000 | 92 |
| CD | 41.903011322 | 37.522361755 | 73.516281128 | PRO | 48 | c2 | 0.0600 | 93 |
| HD1 | 41.081176758 | 37.871139526 | 72.860626221 | PRO | 48 | h | 0.1000 | 94 |
| HD2 | 41.490711212 | 36.761222839 | 74.206848145 | PRO | 48 | h | 0.1000 | 95 |
| C | 44.448692322 | 39.844142914 | 73.044319153 | PRO | 48 | c' | 0.3800 | 96 |
| O | 43.693031311 | 40.225761414 | 72.144134521 | PRO | 48 | o' | -0.3800 | 97 |
| CB | 44.301902771 | 37.304885864 | 73.500595093 | PRO | 48 | c2 | -0.2000 | 98 |
| HB1 | 45.227554321 | 37.245040894 | 72.897834778 | PRO | 48 | h | 0.1000 | 99 |
| HB2 | 44.442562103 | 36.597515106 | 74.341529846 | PRO | 48 | h | 0.1000 | 100 |
| CG | 43.051033020 | 36.925685883 | 72.702011108 | PRO | 48 | c2 | -0.2000 | 101 |
| HG1 | 43.084365845 | 37.389282227 | 71.696128845 | PRO | 48 | h | 0.1000 | 102 |
| HG2 | 42.948661804 | 35.835418701 | 72.555740356 | PRO | 48 | h | 0.1000 | 103 |
| N | 45.700798035 | 40.324272156 | 73.165817261 | GLY | 49 | n | -0.5000 | 104 |
| CA | 46.289730072 | 41.291931152 | 72.184875488 | GLY | 49 | cg | 0.0200 | 105 |
| HN | 46.207077026 | 39.986907959 | 73.991729736 | GLY | 49 | hn | 0.2800 | 106 |
| HA1 | 45.620616913 | 41.460151672 | 71.317451477 | GLY | 49 | h | 0.1000 | 107 |
| HA2 | 46.357063293 | 42.283206940 | 72.672386169 | GLY | 49 | h | 0.1000 | 108 |
| C | 47.682319641 | 40.950855255 | 71.600997925 | GLY | 49 | c' | 0.3800 | 109 |
| O | 48.560806274 | 41.811416626 | 71.601501465 | GLY | 49 | o' | -0.3800 | 110 |
| N | 47.842975616 | 39.718383789 | 71.091018677 | HIS | 50 | n | -0.5000 | 111 |
| HN | 46.947166443 | 39.222202301 | 71.052452087 | HIS | 50 | hn | 0.2800 | 112 |
| CA | 49.020114899 | 39.216835022 | 70.306198120 | HIS | 50 | ca | 0.1200 | 113 |
| HA | 49.324539185 | 38.296161652 | 70.836235046 | HIS | 50 | h | 0.1000 | 114 |
| C | 50.400863647 | 39.993576050 | 70.182189941 | HIS | 50 | c' | 0.3800 | 115 |
| O | 50.733478546 | 40.446022034 | 69.081466675 | HIS | 50 | o' | -0.3800 | 116 |
| CB | 48.455345154 | 38.673969269 | 68.953773499 | HIS | 50 | c2 | -0.2000 | 117 |
| HB1 | 49.238502502 | 38.051708221 | 68.481765747 | HIS | 50 | h | 0.1000 | 118 |
| HB2 | 47.639427185 | 37.951225281 | 69.144165039 | HIS | 50 | h | 0.1000 | 119 |
| CG | 47.954322815 | 39.695293427 | 67.919425964 | HIS | 50 | c5 | 0.1000 | 120 |
| ND1 | 46.759342194 | 40.404747009 | 68.035293579 | HIS | 50 | np | -0.4200 | 121 |
| CE1 | 46.825572968 | 41.133701324 | 66.873245239 | HIS | 50 | c5 | 0.2700 | 122 |
| NE2 | 47.887611389 | 40.964004517 | 66.014495850 | HIS | 50 | np | -0.5000 | 123 |
| CD2 | 48.617565155 | 40.019775391 | 66.717041016 | HIS | 50 | c5 | 0.0100 | 124 |
| HE1 | 46.043247223 | 41.852840424 | 66.655242920 | HIS | 50 | h | 0.1300 | 125 |
| HE2 | 48.092224121 | 41.403381348 | 65.108955383 | HIS | 50 | hn | 0.2800 | 126 |
| HD2 | 49.566501617 | 39.599807739 | 66.396347046 | HIS | 50 | h | 0.1300 | 127 |
| N | 51.278770447 | 40.105480194 | 71.226615906 | PRO | 51 | n | -0.4200 | 128 |
| CA | 52.667034149 | 40.618915558 | 71.077911377 | PRO | 51 | ca | 0.0600 | 129 |
| HA | 52.723186493 | 41.393623352 | 70.287094116 | PRO | 51 | h | 0.1000 | 130 |
| CD | 50.956447601 | 39.767082214 | 72.624496460 | PRO | 51 | c2 | 0.0600 | 131 |
| HD1 | 50.920970917 | 38.670558929 | 72.747383118 | PRO | 51 | h | 0.1000 | 132 |
| HD2 | 49.988422394 | 40.190521240 | 72.947166443 | PRO | 51 | h | 0.1000 | 133 |
| C | 53.707103729 | 39.478122711 | 70.804489136 | PRO | 51 | c' | 0.3800 | 134 |
| O | 53.623699188 | 38.391666412 | 71.391418457 | PRO | 51 | o' | -0.3800 | 135 |
| CB | 52.846694946 | 41.283794403 | 72.455955505 | PRO | 51 | c2 | -0.2000 | 136 |
| HB1 | 53.907955170 | 41.442169189 | 72.729286194 | PRO | 51 | h | 0.1000 | 137 |
| HB2 | 52.373264313 | 42.285846710 | 72.449127197 | PRO | 51 | h | 0.1000 | 138 |
| CG | 52.105823517 | 40.370025635 | 73.440399170 | PRO | 51 | c2 | -0.2000 | 139 |
| HG1 | 52.782051086 | 39.565395355 | 73.789718628 | PRO | 51 | h | 0.1000 | 140 |
| HG2 | 51.753723145 | 40.912036896 | 74.337333679 | PRO | 51 | h | 0.1000 | 141 |
| N | 54.704883575 | 39.729522705 | 69.933471680 | LEU | 52 | n | -0.5000 | 142 |
| CA | 55.791530609 | 36.746330261 | 69.603820801 | LEU | 52 | ca | 0.1200 | 143 |
| HN | 54.653743744 | 40.652229309 | 69.490356445 | LEU | 52 | hn | 0.2800 | 144 |
| HA | 56.479202271 | 39.288208008 | 68.927917480 | LEU | 52 | h | 0.1000 | 145 |
| C | 55.301837921 | 37.525024414 | 68.745040894 | LEU | 52 | c' | 0.3800 | 146 |
| O | 55.637695313 | 37.425930023 | 67.562049866 | LEU | 52 | o' | -0.3800 | 147 |
| CB | 56.671585083 | 38.316761017 | 70.829437256 | LEU | 52 | c2 | -0.2000 | 148 |
| HB1 | 56.036743164 | 37.710464478 | 71.502593994 | LEU | 52 | h | 0.1000 | 149 |
| HB2 | 57.445541382 | 37.602729797 | 70.487434387 | LEU | 52 | h | 0.1000 | 150 |
| CG | 57.363307953 | 39.420749664 | 71.675102234 | LEU | 52 | c1 | -0.1000 | 151 |
| HG | 56.617557526 | 40.200679779 | 71.926834106 | LEU | 52 | h | 0.1000 | 152 |
| CD1 | 57.875057220 | 38.833915710 | 73.004547119 | LEU | 52 | c3 | -0.3000 | 153 |
| HD11 | 58.353130341 | 39.601875305 | 73.642135620 | LEU | 52 | h | 0.1000 | 154 |
| HD12 | 57.048751831 | 38.403957367 | 73.601577759 | LEU | 52 | h | 0.1000 | 155 |
| HD13 | 58.618564606 | 38.028480530 | 72.852462769 | LEU | 52 | h | 0.1000 | 156 |
| CD2 | 58.531608582 | 40.085853577 | 70.927200317 | LEU | 52 | c3 | -0.3000 | 157 |
| HD21 | 59.028976440 | 40.857166290 | 71.545303345 | LEU | 52 | h | 0.1000 | 158 |
| HD22 | 59.309509277 | 39.355545044 | 70.634819031 | LEU | 52 | h | 0.1000 | 159 |
| HD23 | 58.192760468 | 40.592193604 | 70.005569458 | LEU | 52 | h | 0.1000 | 160 |
| N | 54.534648895 | 36.601863861 | 69.354270935 | ALA | 53 | n | -0.5000 | 161 |
| CA | 53.940563202 | 35.420303345 | 68.673507690 | ALA | 53 | ca | 0.1200 | 162 |
| HN | 54.159572601 | 36.958141327 | 70.246543884 | ALA | 53 | hn | 0.2800 | 163 |
| HA | 53.600482941 | 35.753322601 | 67.671340942 | ALA | 53 | h | 0.1000 | 164 |
| C | 52.639778137 | 34.883575439 | 69.383995056 | ALA | 53 | c' | 0.3800 | 165 |
| O | 51.628326416 | 34.818698883 | 68.677047729 | ALA | 53 | o' | -0.3800 | 166 |
| CB | 55.008785248 | 34.330768585 | 68.423698425 | ALA | 53 | c3 | -0.3000 | 167 |
| HB1 | 54.582756042 | 33.460170746 | 67.892036438 | ALA | 53 | h | 0.1000 | 168 |
| HB2 | 55.828662872 | 34.711517334 | 67.787284851 | ALA | 53 | h | 0.1000 | 169 |
| HB3 | 55.479701996 | 33.961406708 | 69.351325989 | ALA | 53 | h | 0.1000 | 170 |
| N | 52.555103302 | 34.484893799 | 70.698265076 | PRO | 54 | n | -0.4200 | 171 |
| CA | 51.304950714 | 33.917499542 | 71.286506653 | PRO | 54 | ca | 0.0600 | 172 |
| HA | 50.878768921 | 33.172523499 | 70.584587097 | PRO | 54 | h | 0.1000 | 173 |
| CD | 53.705833435 | 34.435420990 | 71.626182556 | PRO | 54 | c2 | 0.0600 | 174 |
| HD1 | 54.215991974 | 35.409980774 | 71.739936829 | PRO | 54 | h | 0.1000 | 175 |
| HD2 | 54.453365326 | 33.693523407 | 71.288909912 | PRO | 54 | h | 0.1000 | 176 |
| C | 50.177021027 | 34.945980072 | 71.642837524 | PRO | 54 | c' | 0.3800 | 177 |
| O | 50.377983093 | 36.164409637 | 71.677795410 | PRO | 54 | o' | -0.3800 | 178 |
| CB | 51.867893219 | 33.173828125 | 72.519187927 | PRO | 54 | c2 | -0.2000 | 179 |
| HB1 | 51.135505676 | 33.051830292 | 73.340660095 | PRO | 54 | h | 0.1000 | 180 |
| HB2 | 52.181377411 | 32.150829315 | 72.232276917 | PRO | 54 | h | 0.1000 | 181 |
| CG | 53.087123871 | 33.989192963 | 72.949317932 | PRO | 54 | c2 | -0.2000 | 182 |
| HG1 | 52.768703461 | 34.871643066 | 73.538475037 | PRO | 54 | h | 0.1000 | 183 |
| HG2 | 53.791828156 | 33.414070129 | 73.579086304 | PRO | 54 | h | 0.1000 | 184 |
| N | 48.977436066 | 34.414421082 | 71.936706543 | GLY | 55 | n | -0.5000 | 185 |
| CA | 47.822620392 | 35.225021362 | 72.404747009 | GLY | 55 | cg | 0.0200 | 186 |
| HN | 48.958133698 | 33.389709473 | 71.929512024 | GLY | 55 | hn | 0.2800 | 187 |
| HA1 | 47.830284119 | 36.238574982 | 71.963676453 | GLY | 55 | h | 0.1000 | 188 |
| HA2 | 46.896526337 | 34.772380829 | 72.004432678 | GLY | 55 | h | 0.1000 | 189 |
| C | 47.670829773 | 35.263648987 | 73.950416565 | GLY | 55 | c' | 0.3800 | 190 |
| O | 47.247509003 | 34.242198944 | 74.498336792 | GLY | 55 | o' | -0.3800 | 191 |
| N | 47.956153870 | 36.372848511 | 74.696281433 | PRO | 56 | n | -0.4200 | 192 |
| CA | 47.830066681 | 36.396587372 | 76.179130554 | PRO | 56 | ca | 0.0600 | 193 |
| HA | 48.225147247 | 35.457695007 | 76.619560242 | PRO | 56 | h | 0.1000 | 194 |
| CD | 48.653686523 | 37.556163788 | 74.163940430 | PRO | 56 | c2 | 0.0600 | 195 |
| HD1 | 48.108860016 | 38.040233612 | 73.332565308 | PRO | 56 | h | 0.1000 | 196 |
| HD2 | 49.652721405 | 37.256084442 | 73.799308777 | PRO | 56 | h | 0.1000 | 197 |
| C | 46.361907959 | 36.604877472 | 76.668052673 | PRO | 56 | c' | 0.3800 | 198 |
| O | 45.890090942 | 37.732730865 | 76.845039368 | PRO | 56 | o' | -0.3800 | 199 |
| CB | 48.804897308 | 37.531764984 | 76.560951233 | PRO | 56 | c2 | -0.2000 | 200 |
| HB1 | 48.542221069 | 38.034294128 | 77.511970520 | PRO | 56 | h | 0.1000 | 201 |
| HB2 | 49.825592041 | 37.124542236 | 76.697402954 | PRO | 56 | h | 0.1000 | 202 |
| CG | 48.782566071 | 38.488700867 | 75.368530273 | PRO | 56 | c2 | -0.2000 | 203 |
| HG1 | 47.903289795 | 39.158111572 | 75.434867859 | PRO | 56 | h | 0.1000 | 204 |
| HG2 | 49.679012299 | 39.133480072 | 75.321792603 | PRO | 56 | h | 0.1000 | 205 |
| N | 45.650573730 | 35.488880157 | 76.896896362 | HIS | 57 | n | -0.5000 | 206 |
| HN | 46.046119690 | 34.657379150 | 76.439048767 | HIS | 57 | hn | 0.2800 | 207 |
| CA | 44.244419098 | 35.504474640 | 77.387596130 | HIS | 57 | ca | 0.1200 | 208 |
| HA | 43.667560577 | 36.207649231 | 76.759368896 | HIS | 57 | h | 0.1000 | 209 |
| C | 44.173530579 | 35.943927765 | 78.901000977 | HIS | 57 | c' | 0.3800 | 210 |
| O | 44.750030518 | 35.234142303 | 79.736030579 | HIS | 57 | o' | -0.3800 | 211 |
| CB | 43.610416412 | 34.095542908 | 77.185058594 | HIS | 57 | c2 | -0.2000 | 212 |
| HB1 | 44.270858765 | 33.323795319 | 77.623748779 | HIS | 57 | h | 0.1000 | 213 |
| HB2 | 42.689029694 | 34.034877777 | 77.796188354 | HIS | 57 | h | 0.1000 | 214 |
| CG | 43.250400543 | 33.671833038 | 75.751731873 | HIS | 57 | c5 | 0.1000 | 215 |
| ND1 | 44.116428375 | 33.723644257 | 74.666069031 | HIS | 57 | np | -0.4200 | 216 |
| CE1 | 43.325973511 | 33.139041901 | 73.713264465 | HIS | 57 | c5 | 0.2700 | 217 |
| NE2 | 42.066158295 | 32.716590881 | 74.036811829 | HIS | 57 | np | -0.5000 | 218 |
| CD2 | 42.045005798 | 33.048488617 | 75.379264832 | HIS | 57 | c5 | 0.0100 | 219 |
| HE1 | 43.712070465 | 33.001682281 | 72.711807251 | HIS | 57 | h | 0.1300 | 220 |
| HE2 | 41.370864868 | 32.225383759 | 73.464263916 | HIS | 57 | hn | 0.2800 | 221 |
| HD2 | 41.236827850 | 32.815635681 | 76.058380127 | HIS | 57 | h | 0.1300 | 222 |
| N | 43.513858795 | 37.068405151 | 79.318038940 | PRO | 58 | n | -0.4200 | 223 |
| CA | 43.593978882 | 37.569736481 | 80.719970703 | PRO | 58 | ca | 0.0600 | 224 |
| HA | 44.657642365 | 37.630668640 | 81.026939392 | PRO | 58 | h | 0.1000 | 225 |
| CD | 42.833599091 | 38.010932922 | 78.407653809 | PRO | 58 | c2 | 0.0600 | 226 |
| HD1 | 42.093353271 | 37.516807556 | 77.751007080 | PRO | 58 | h | 0.1000 | 227 |
| HD2 | 43.569110870 | 38.527889252 | 77.758674622 | PRO | 58 | h | 0.1000 | 228 |
| C | 42.805988312 | 36.695770264 | 81.747009277 | PRO | 58 | c' | 0.3800 | 229 |
| O | 41.569808960 | 36.693984985 | 81.769706726 | PRO | 58 | o' | -0.3800 | 230 |
| CB | 43.072513580 | 39.016571045 | 80.579101563 | PRO | 58 | c2 | -0.2000 | 231 |
| HB1 | 42.559799194 | 39.392253876 | 81.485702515 | PRO | 58 | h | 0.1000 | 232 |
| HB2 | 43.920654297 | 39.706352234 | 80.398040771 | PRO | 58 | h | 0.1000 | 233 |
| CG | 42.156440735 | 38.999496460 | 79.353309631 | PRO | 58 | c2 | -0.2000 | 234 |
| HG1 | 41.151851654 | 38.629653931 | 79.634307861 | PRO | 58 | h | 0.1000 | 235 |
| HG2 | 42.023620605 | 39.998752594 | 78.896965027 | PRO | 58 | h | 0.1000 | 236 |
| N | 43.540618896 | 35.961261749 | 82.605430603 | ALA | 59 | n | -0.5000 | 237 |
| HN | 44.537330627 | 35.932937622 | 82.365699768 | ALA | 59 | hn | 0.2800 | 238 |
| CA | 42.949653625 | 34.946208954 | 83.526855469 | ALA | 59 | ca | 0.1200 | 239 |
| HA | 42.197692871 | 34.355514526 | 82.965911865 | ALA | 59 | h | 0.1000 | 240 |
| C | 42.208984375 | 35.488883972 | 84.803985596 | ALA | 59 | c' | 0.3800 | 241 |
| O | 42.496433258 | 35.127353668 | 85.948333740 | ALA | 59 | o' | -0.3800 | 242 |
| CB | 44.107444763 | 33.975612640 | 83.839767456 | ALA | 59 | c3 | -0.3000 | 243 |
| HB1 | 43.761249542 | 33.130538940 | 84.463287354 | ALA | 59 | h | 0.1000 | 244 |
| HB2 | 44.544620514 | 33.531585693 | 82.924507141 | ALA | 59 | h | 0.1000 | 245 |
| HB3 | 44.925910950 | 34.467308044 | 84.399597168 | ALA | 59 | h | 0.1000 | 246 |
| N | 41.192062378 | 36.323741913 | 84.559051514 | ALA | 60 | n | -0.5000 | 247 |
| HN | 41.124549866 | 36.554992676 | 83.555343628 | ALA | 60 | hn | 0.2800 | 248 |
| CA | 40.203086853 | 36.790748596 | 85.562988281 | ALA | 60 | ca | 0.1200 | 249 |
| HA | 40.023948669 | 35.965785980 | 86.283721924 | ALA | 60 | h | 0.1000 | 250 |
| C | 38.823528290 | 37.027305603 | 84.846977234 | ALA | 60 | c' | 0.3800 | 251 |
| O | 37.897804260 | 36.283885956 | 85.186340332 | ALA | 60 | o' | -0.3800 | 252 |
| CB | 40.756233215 | 37.971691132 | 86.389617920 | ALA | 60 | c3 | -0.3000 | 253 |
| HB1 | 40.004146576 | 38.357006073 | 87.102043152 | ALA | 60 | h | 0.1000 | 254 |
| HB2 | 41.631908417 | 37.656120300 | 86.987319946 | ALA | 60 | h | 0.1000 | 255 |
| HB3 | 41.090072632 | 38.818138123 | 85.765472412 | ALA | 60 | h | 0.1000 | 256 |
| N | 38.616340637 | 37.921970367 | 83.823921204 | PRO | 61 | n | -0.4200 | 257 |
| CA | 37.403762817 | 37.874496460 | 82.948486328 | PRO | 61 | ca | 0.0600 | 258 |
| HA | 36.491020203 | 37.824863434 | 83.573593140 | PRO | 61 | h | 0.1000 | 259 |
| CD | 39.556003571 | 39.003845215 | 83.459777832 | PRO | 61 | c2 | 0.0600 | 260 |
| HD1 | 40.594814301 | 38.658184052 | 83.304046631 | PRO | 61 | h | 0.1000 | 261 |
| HD2 | 39.571029663 | 39.778694153 | 84.250648499 | PRO | 61 | h | 0.1000 | 262 |
| C | 37.275394440 | 36.712963104 | 81.892074585 | PRO | 61 | c' | 0.3800 | 263 |
| O | 36.266387939 | 36.670307159 | 81.183494568 | PRO | 61 | o' | -0.3800 | 264 |
| CB | 37.473857880 | 39.266963959 | 82.286987305 | PRO | 61 | c2 | -0.2000 | 265 |
| HB1 | 36.949714661 | 39.321308136 | 81.312759399 | PRO | 61 | h | 0.1000 | 266 |
| HB2 | 36.985511780 | 40.017913818 | 82.938987732 | PRO | 61 | h | 0.1000 | 267 |
| CG | 38.968631744 | 39.571613312 | 82.168632507 | PRO | 61 | c2 | -0.2000 | 268 |
| HG1 | 39.397396088 | 39.046298981 | 81.292793274 | PRO | 61 | h | 0.1000 | 269 |
| HG2 | 39.180202484 | 40.649600983 | 82.039207458 | PRO | 61 | h | 0.1000 | 270 |
| N | 38.242324829 | 35.777751923 | 81.785034180 | SER | 62 | n | -0.5000 | 271 |
| CA | 38.186004639 | 34.624855042 | 80.844123840 | SER | 62 | ca | 0.1200 | 272 |
| HN | 39.035541534 | 35.927360535 | 82.416992188 | SER | 62 | hn | 0.2800 | 273 |
| HA | 37.921787262 | 35.006183624 | 79.841125488 | SER | 62 | h | 0.1000 | 274 |
| C | 37.145660400 | 33.532897949 | 81.261909485 | SER | 62 | c' | 0.3800 | 275 |
| O | 37.466091156 | 32.626064301 | 82.041137695 | SER | 62 | o' | -0.3800 | 276 |
| CB | 39.620265961 | 34.048240662 | 80.725196838 | SER | 62 | c2 | -0.1700 | 277 |
| HB1 | 39.660293579 | 33.306644440 | 79.904281616 | SER | 62 | h | 0.1000 | 278 |
| HB2 | 40.349685669 | 34.832687378 | 80.442802429 | SER | 62 | h | 0.1000 | 279 |
| OG | 40.032703400 | 33.414188385 | 81.938880920 | SER | 62 | oh | -0.3800 | 280 |
| HG | 39.252223969 | 32.931293488 | 82.256263733 | SER | 62 | ho | 0.3500 | 281 |
| N | 35.902244568 | 33.647918701 | 80.764541626 | SER | 63 | n | -0.5000 | 282 |
| CA | 34.747528076 | 32.874465942 | 81.297317505 | SER | 63 | ca | 0.1200 | 283 |
| HN | 35.768447876 | 34.503852844 | 80.205200195 | SER | 63 | hn | 0.2800 | 284 |
| HA | 35.064254761 | 32.265518188 | 82.170570374 | SER | 63 | h | 0.1000 | 285 |
| C | 34.106758118 | 31.936998367 | 80.231674194 | SER | 63 | c' | 0.3800 | 286 |
| O | 33.716896057 | 32.367130280 | 79.142120361 | SER | 63 | o' | -0.3800 | 287 |
| CB | 33.716815948 | 33.889484406 | 81.843544006 | SER | 63 | c2 | -0.1700 | 288 |
| HB1 | 34.199871063 | 34.571384430 | 82.572105408 | SER | 63 | h | 0.1000 | 289 |
| HB2 | 33.328830719 | 34.543502808 | 81.036796570 | SER | 63 | h | 0.1000 | 290 |
| OG | 32.634590149 | 33.222091675 | 82.496467590 | SER | 63 | oh | -0.3800 | 291 |
| HG | 32.159793854 | 32.710407257 | 81.832328796 | SER | 63 | ho | 0.3500 | 292 |
| N | 33.914913177 | 30.658897400 | 80.588378906 | TRP | 64 | n | -0.5000 | 293 |
| CA | 33.112319946 | 29.694124222 | 79.783546448 | TRP | 64 | ca | 0.1200 | 294 |
| HN | 34.221500397 | 30.422899246 | 81.538955688 | TRP | 64 | hn | 0.2800 | 295 |
| HA | 33.404731750 | 29.812168121 | 78.721931458 | TRP | 64 | h | 0.1000 | 296 |
| C | 31.573041916 | 29.961977005 | 79.883049011 | TRP | 64 | c' | 0.3800 | 297 |
| o | 30.996980667 | 29.940891266 | 80.975959778 | TRP | 64 | o' | -0.3800 | 298 |
| CB | 33.525466919 | 26.235471725 | 80.142707825 | TRP | 64 | c2 | -0.2000 | 299 |
| HB1 | 32.950366974 | 27.534402847 | 79.508041382 | TRP | 64 | h | 0.1000 | 300 |
| HB2 | 34.571674347 | 28.078489304 | 79.816658020 | TRP | 64 | h | 0.1000 | 301 |
| CG | 33.405326843 | 27.783784866 | 81.611763000 | TRP | 64 | c5 | 0.0000 | 302 |
| CD1 | 32.267101288 | 27.214570999 | 82.221214294 | TRP | 64 | c5 | 0.0100 | 303 |
| NE1 | 32.481933594 | 26.953943253 | 83.590408325 | TRP | 64 | hp | -0.5000 | 304 |
| CE2 | 33.781982422 | 27.378627777 | 83.812339783 | TRP | 64 | c5 | 0.1100 | 305 |
| CD2 | 34.355152130 | 27.881061554 | 82.617965698 | TRP | 64 | c5 | 0.0000 | 306 |
| HD1 | 31.332025528 | 27.036033630 | 81.708480835 | TRP | 64 | h | 0.1000 | 307 |
| HE1 | 31.820940018 | 26.578481674 | 84.279945374 | TRP | 64 | n | 0.2800 | 308 |
| CE3 | 35.681034088 | 28.394184113 | 82.615905762 | TRP | 64 | cp | -0.1000 | 309 |
| HE3 | 36.128986359 | 28.784986496 | 81.714393616 | TRP | 64 | h | 0.1000 | 310 |
| CZ3 | 36.396430969 | 28.387191772 | 82.815704346 | TRP | 64 | cp | -0.1000 | 311 |
| HZ3 | 37.405311584 | 28.776082993 | 83.832160950 | TRP | 64 | h | 0.1000 | 312 |
| CH2 | 35.830604553 | 27.888952255 | 84.994010925 | TRP | 64 | cp | -0.1000 | 313 |
| HH2 | 36.410472870 | 27.896844864 | 85.906951904 | TRP | 64 | h | 0.1000 | 314 |
| CZ2 | 34.527233124 | 27.382776260 | 85.014289856 | TRP | 64 | cp | -0.1000 | 315 |
| HZ2 | 34.097515106 | 27.006088257 | 85.929389954 | TRP | 64 | h | 0.1000 | 316 |
| N | 30.921329498 | 30.232547760 | 78.740600586 | GLY | 65 | n | -0.5000 | 317 |
| CA | 29.460748672 | 30.504768372 | 78.692192078 | GLY | 65 | cg | 0.0200 | 318 |
| HN | 31.520374298 | 30.302478790 | 77.901519775 | GLY | 65 | hn | 0.2800 | 319 |
| HA1 | 29.073087692 | 30.896234512 | 79.650825500 | GLY | 65 | h | 0.1000 | 320 |
| HA2 | 29.288171768 | 31.333106995 | 77.981094360 | GLY | 65 | h | 0.1000 | 321 |
| C | 28.633579254 | 29.293350220 | 78.197364807 | GLY | 65 | c' | 0.3800 | 322 |
| O | 28.566907883 | 29.137302399 | 76.975486755 | GLY | 65 | o' | -0.3800 | 323 |
| N | 27.989013672 | 28.429246902 | 79.038352966 | PRO | 66 | n | -0.4200 | 324 |
| CA | 27.282257080 | 27.212890625 | 78.546752930 | PRO | 66 | ca | 0.0600 | 325 |
| HA | 27.989650726 | 26.634012222 | 77.917152405 | PRO | 66 | h | 0.1000 | 326 |
| CD | 28.016592026 | 28.532529831 | 80.511337280 | PRO | 66 | c2 | 0.0600 | 327 |
| HD1 | 27.731332779 | 29.536006927 | 80.880874634 | PRO | 66 | h | 0.1000 | 328 |
| HD2 | 29.027824402 | 28.301166534 | 80.897590637 | PRO | 66 | h | 0.1000 | 329 |
| C | 25.977466583 | 27.479314804 | 77.725341797 | PRO | 66 | c' | 0.3800 | 330 |
| O | 25.217950821 | 28.417282104 | 77.982574463 | PRO | 66 | o' | -0.3800 | 331 |
| CB | 27.045602798 | 26.422634125 | 79.851196289 | PRO | 66 | c2 | -0.2000 | 332 |
| HB1 | 26.132562637 | 25.797079086 | 79.827728271 | PRO | 66 | h | 0.1000 | 333 |
| HB2 | 27.890687943 | 25.729280472 | 80.029365540 | PRO | 66 | h | 0.1000 | 334 |
| CG | 27.003501892 | 27.477243423 | 80.958015442 | PRO | 66 | c2 | -0.2000 | 335 |
| HG1 | 25.990892410 | 27.921483994 | 81.014678955 | PRO | 66h | h | 0.1000 | 336 |
| HG2 | 27.232566833 | 27.061700821 | 81.956459045 | PRO | 66 | h | 0.1000 | 337 |
| N | 25.734319687 | 26.626403809 | 76.719802856 | ARG+ | 67 | n | -0.5000 | 338 |
| CA | 24.603988647 | 26.793025970 | 75.767257690 | ARG+ | 67 | ca | 0.1200 | 339 |
| HN | 26.386735916 | 25.841371536 | 76.649803162 | ARG+ | 67 | hn | 0.2800 | 340 |
| HA | 24.496238708 | 27.874828339 | 75.561668396 | ARG+ | 67 | h | 0.1000 | 341 |
| C | 23.227464676 | 26.224872589 | 76.267372131 | ARG+ | 67 | c' | 0.3800 | 342 |
| O | 23.178310394 | 25.034952164 | 76.603759766 | ARG+ | 67 | o' | -0.3800 | 343 |
| CB | 24.990058899 | 26.165229797 | 74.398826599 | ARG+ | 67 | c2 | -0.2000 | 344 |
| HB1 | 24.135663986 | 26.318639755 | 73.709175110 | ARG+ | 67 | h | 0.1100 | 345 |
| HB2 | 25.787433624 | 26.779323578 | 73.940032959 | ARG+ | 67 | h | 0.1100 | 346 |
| CG | 25.439929962 | 24.676465988 | 74.361564636 | ARG+ | 67 | c2 | -0.2000 | 347 |
| HG1 | 26.546255112 | 24.646316528 | 74.415458679 | ARG+ | 67 | h | 0.1300 | 348 |
| HG2 | 25.092346191 | 24.131168365 | 75.261718750 | ARG+ | 67 | h | 0.1300 | 349 |
| CD | 24.934387207 | 23.941221237 | 73.112297058 | ARG+ | 67 | c2 | -0.0900 | 350 |
| HD1 | 23.838283539 | 23.774566650 | 73.188652039 | ARG+ | 67 | h | 0.1300 | 351 |
| HD2 | 25.070211411 | 24.585262299 | 72.220893860 | ARG+ | 67 | h | 0.1300 | 352 |
| NE | 25.665744781 | 22.657058716 | 72.968780518 | ARG+ | 67 | n1 | -0.5000 | 353 |
| HE | 26.251846313 | 22.313375473 | 73.731925964 | ARG+ | 67 | hn | 0.3600 | 354 |
| CZ | 25.689014435 | 21.902687073 | 71.871635437 | ARG+ | 67 | cr | 0.4500 | 355 |
| NH1 | 26.493299484 | 20.879484177 | 71.859733582 | ARG+ | 67 | n2 | -0.5000 | 356 |
| HH11 | 27.072675705 | 20.740955353 | 72.690315247 | ARG+ | 67 | hn | 0.3600 | 357 |
| HH12 | 26.520929337 | 20.319580078 | 71.006805420 | ARG+ | 67 | hn | 0.3600 | 358 |
| NH2 | 24.956668854 | 22.117029190 | 70.805320740 | ARG+ | 67 | n2 | -0.5000 | 359 |
| HH21 | 25.030595779 | 21.456792831 | 70.033142090 | ARG+ | 67 | hn | 0.3600 | 360 |
| HH22 | 24.266489029 | 22.916227341 | 70.850784302 | ARG+ | 67 | hn | 0.3600 | 361 |
| N | 22.080270767 | 26.971176147 | 76.244255066 | PRO | 68 | n | -0.4200 | 362 |
| CA | 20.743734360 | 26.358839035 | 76.485237122 | PRO | 68 | ca | 0.0600 | 363 |
| HA | 20.817556381 | 25.605155945 | 77.294357300 | PRO | 68 | h | 0.1000 | 364 |
| CD | 22.076143265 | 28.448001862 | 76.342918396 | PRO | 68 | c2 | 0.0600 | 365 |
| HD1 | 22.539228439 | 28.949869156 | 75.469612122 | PRO | 68 | h | 0.1000 | 366 |
| HD2 | 22.632146835 | 28.776126862 | 77.244499207 | PRO | 68 | h | 0.1000 | 367 |
| C | 20.182382584 | 25.586324692 | 75.240180969 | PRO | 68 | c' | 0.3800 | 368 |
| O | 20.420539856 | 24.381649017 | 75.139877319 | PRO | 68 | o' | -0.3800 | 369 |
| CB | 19.948141098 | 27.549791336 | 77.062515259 | PRO | 68 | c2 | -0.2000 | 370 |
| HB1 | 18.858430862 | 27.490163803 | 76.877128601 | PRO | 68 | h | 0.1000 | 371 |
| HB2 | 20.066789627 | 27.567596436 | 78.163002014 | PRO | 68 | h | 0.1000 | 372 |
| CG | 20.592071533 | 28.804227829 | 76.467758179 | PRO | 68 | c2 | -0.2000 | 373 |
| HG1 | 20.158363342 | 29.031393051 | 75.478172302 | PRO | 68 | h | 0.1000 | 374 |
| HG2 | 20.428398132 | 29.704229355 | 77.092338562 | PRO | 68 | h | 0.1000 | 375 |
| N | 19.458055496 | 26.229068756 | 74.300010681 | ARG+ | 69 | n | -0.5000 | 376 |
| CA | 18.893756866 | 25.542047501 | 73.096710205 | ARG+ | 69 | ca | 0.1200 | 377 |
| HN | 19.221296310 | 27.198928833 | 74.529014587 | ARG+ | 69 | hn | 0.2800 | 378 |
| HA | 19.667610168 | 24.872461319 | 72.660797119 | ARG+ | 69 | h | 0.1000 | 379 |
| C | 18.514461517 | 26.608341217 | 72.012504578 | ARG+ | 69 | c' | 0.3800 | 380 |
| O | 17.383218765 | 27.099323273 | 72.036094666 | ARG+ | 69 | o' | -0.3800 | 381 |
| CB | 17.681777954 | 24.654710770 | 73.539657593 | ARG+ | 69 | c2 | -0.2000 | 382 |
| HB1 | 18.011884689 | 23.935596466 | 74.315147400 | ARG+ | 69 | h | 0.1100 | 383 |
| HB2 | 16.954420090 | 25.310214996 | 74.061965942 | ARG+ | 69 | h | 0.1100 | 384 |
| CG | 16.946891785 | 23.862810135 | 72.427490234 | ARG+ | 69 | c2 | -0.2000 | 385 |
| HG1 | 16.649517059 | 24.551628113 | 71.612152100 | ARG+ | 69 | h | 0.1300 | 386 |
| HG2 | 17.638776779 | 23.127803802 | 71.965805054 | ARG+ | 69 | h | 0.1300 | 387 |
| CD | 15.688191414 | 23.166488647 | 72.975959778 | ARG+ | 69 | c2 | -0.0900 | 388 |
| HD1 | 15.980383873 | 22.365550995 | 73.686569214 | ARG+ | 69 | h | 0.1300 | 389 |
| HD2 | 15.090404510 | 23.898859024 | 73.554351807 | ARG+ | 69 | h | 0.1300 | 390 |
| NE | 14.889810562 | 22.612804413 | 71.848815918 | ARG+ | 69 | n1 | -0.5000 | 391 |
| NE | 15.272388458 | 22.616128922 | 70.898582458 | ARG+ | 69 | hn | 0.3600 | 392 |
| CZ | 13.644455910 | 22.143890381 | 71.942466736 | ARG+ | 69 | cr | 0.4500 | 393 |
| NH1 | 13.048615456 | 21.755460739 | 70.850708008 | ARG+ | 69 | n2 | -0.5000 | 394 |
| HH11 | 13.576630592 | 21.832328796 | 69.979103088 | ARG+ | 69 | hn | 0.3600 | 395 |
| HH12 | 12.090744019 | 21.411947250 | 70.936080933 | ARG+ | 69 | hn | 0.3600 | 396 |
| NH2 | 12.989639282 | 22.056533813 | 73.074882507 | ARG+ | 69 | n2 | -0.5000 | 397 |
| HH21 | 12.033639908 | 21.696094513 | 73.066261292 | ARG+ | 69 | hn | 0.3600 | 398 |
| HH22 | 13.529273987 | 22.372974396 | 73.883934021 | ARG+ | 69 | hn | 0.3600 | 399 |
| N | 19.436628342 | 26.928932190 | 71.074501038 | ARG+ | 70 | n | -0.5000 | 400 |
| CA | 19.223009109 | 27.811206818 | 69.878326416 | ARG+ | 70 | ca | 0.1200 | 401 |
| HN | 20.357131958 | 26.451332092 | 71.165985107 | ARG+ | 70 | hn | 0.2800 | 402 |
| HA | 19.087514877 | 27.065124512 | 69.071128845 | ARG+ | 70 | h | 0.1000 | 403 |
| C | 20.512538910 | 28.575149536 | 69.398536682 | ARG+ | 70 | c' | 0.3800 | 404 |
| O | 20.872812271 | 28.468791962 | 68.228363037 | ARG+ | 70 | o' | -0.3800 | 405 |
| CB | 17.935552597 | 28.697717667 | 69.732887268 | ARG+ | 70 | c2 | -0.2000 | 406 |
| HB1 | 17.889257431 | 29.085472107 | 68.694030762 | ARG+ | 70 | h | 0.1100 | 407 |
| HB2 | 17.053388596 | 28.033058167 | 69.807891846 | ARG+ | 70 | h | 0.1100 | 408 |
| CG | 17.775762558 | 29.883768082 | 70.717842102 | ARG+ | 70 | c2 | -0.2000 | 409 |
| HG1 | 18.004568100 | 29.546030045 | 71.747383118 | ARG+ | 70 | h | 0.1300 | 410 |
| HG2 | 18.540782928 | 30.651863098 | 70.495643616 | ARG+ | 70 | h | 0.1300 | 411 |
| CD | 16.368116379 | 30.502414703 | 70.693214417 | ARG+ | 70 | c2 | -0.0900 | 412 |
| HD1 | 16.095691681 | 30.812221527 | 69.664886475 | ARG+ | 70 | h | 0.1300 | 413 |
| HD2 | 15.630161285 | 29.711160660 | 70.937812805 | ARG+ | 70 | h | 0.1300 | 414 |
| NE | 16.255954742 | 31.590759277 | 71.711013794 | ARG+ | 70 | n1 | -0.5000 | 415 |
| HE | 16.253637314 | 31.350564957 | 72.706428528 | ARG+ | 70 | hn | 0.3600 | 416 |
| CZ | 16.144437790 | 32.900745392 | 71.464561462 | ARG+ | 70 | cr | 0.4500 | 417 |
| NH1 | 16.055492401 | 33.712890625 | 72.481109619 | ARG+ | 70 | n2 | -0.5000 | 418 |
| HH11 | 16.071464539 | 33.294216156 | 73.413330078 | ARG+ | 70 | hn | 0.3600 | 419 |
| HH12 | 15.975571632 | 34.708621979 | 72.277374268 | ARG+ | 70 | hn | 0.3600 | 420 |
| NH2 | 16.120351791 | 33.420074463 | 70.259872437 | ARG+ | 70 | n2 | -0.5000 | 421 |
| HH21 | 16.025018692 | 34.432674408 | 70.167800903 | ARG+ | 70 | hn | 0.3600 | 422 |
| HH22 | 16.187112808 | 32.736862183 | 69.505996704 | ARG+ | 70 | hn | 0.3600 | 423 |
| N | 21.115812302 | 29.562902451 | 70.071769714 | TYRC | 71 | n | -0.5000 | 424 |
| HN | 21.515977859 | 30.157514572 | 69.338050842 | TYRC | 71 | hn | 0.2800 | 425 |
| CA | 22.034273148 | 29.314456940 | 71.218978882 | TYRC | 71 | ca | 0.1200 | 426 |
| HA | 22.671009064 | 28.444923401 | 70.976676941 | TYRC | 71 | h | 0.1000 | 427 |
| C | 21.352920532 | 28.953493118 | 72.563385010 | TYRC | 71 | c' | 0.4100 | 428 |
| OXT | 20.392858505 | 29.553161621 | 73.048652649 | TYRC | 71 | o' | -0.3800 | 429 |
| O | 21.928325653 | 27.853042603 | 73.145797729 | TYRC | 71 | oh | -0.3800 | 430 |
| HO | 21.429273605 | 27.558662415 | 73.909782410 | TYRC | 71 | ho | 0.3500 | 431 |
| CB | 22.969152451 | 30.555358887 | 71.361984253 | TYRC | 71 | c2 | -0.2000 | 432 |
| HB1 | 22.368267059 | 31.487627029 | 71.355415344 | TYRC | 71 | h | 0.1000 | 433 |
| HB2 | 23.401992798 | 30.559690475 | 72.381835938 | TYRC | 71 | h | 0.1000 | 434 |
| CG | 24.144546509 | 30.666173935 | 70.352111816 | TYRC | 71 | cp | 0.0000 | 435 |
| CD1 | 23.927837372 | 31.126276016 | 69.044418335 | TYRC | 71 | cp | -0.1000 | 436 |
| HD1 | 22.944635391 | 31.424867630 | 68.717597961 | TYRC | 71 | h | 0.1000 | 437 |
| CE1 | 24.987041473 | 31.212265015 | 68.143028259 | TYRC | 71 | cp | -0.1000 | 438 |
| HE1 | 24.819047928 | 31.555503845 | 67.131973267 | TYRC | 71 | h | 0.1000 | 439 |
| CZ | 26.273118973 | 30.861675262 | 68.542274475 | TYRC | 71 | cp | 0.0300 | 440 |
| OH | 27.314481735 | 30.957365036 | 67.652267456 | TYRC | 71 | oh | -0.3800 | 441 |
| HH | 26.980180740 | 31.236070633 | 66.796859741 | TYRC | 71 | ho | 0.3500 | 442 |
| CE2 | 26.504697800 | 30.422637939 | 69.841377258 | TYRC | 71 | cp | -0.1000 | 443 |
| HE2 | 27.503232956 | 30.148866653 | 70.140815735 | TYRC | 71 | h | 0.1000 | 444 |
| CO2 | 25.447391510 | 30.325349808 | 70.743820190 | TYRC | 71 | cp | -0.1000 | 445 |
| HD2 | 25.652494431 | 29.968608856 | 71.745338440 | TYRC | 71 | h | 0.1000 | 430 |

| | | | | | | | | |
|---|---|---|---|---|---|---|---|---|
| * La numérotation des aminoacides est décalée de moins 6 par rapport à la séquence SEQ ID N° 1. | | | | | | | | |

**TABLEAU N° 2 ***

| Nom de l'atome | x | y | z | nom et n° du résidu | | type, charge et n° de l'atome | | |
|---|---|---|---|---|---|---|---|---|
| N | 24.753738403 | 26.435615500 | 68.244300842 | CYSn | 40 | n3 | -0.5000 | 1 |
| CA | 24.503000259 | 26.356292725 | 69.707687378 | CYSn | 40 | ca | 0.1200 | 2 |
| HN1 | 23.861560822 | 26.429992676 | 67.734397888 | CYSn | 40 | hn | 0.1400 | 3 |
| HN2 | 25.250690460 | 25.603437424 | 67.909477234 | CYSn | 40 | hn | 0.1400 | 4 |
| HA | 23.890571594 | 27.247760773 | 69.940788269 | CYSn | 40 | h | 0.1000 | 5 |
| C | 25.747190475 | 26.505004883 | 70.632949629 | CYSn | 40 | c' | 0.3800 | 6 |
| O | 25.611124039 | 27.204542160 | 71.634971619 | CYSn | 40 | o' | -0.3800 | 7 |
| CB | 23.602088928 | 25.125436783 | 69.979545593 | CYSn | 40 | c2 | -0.3000 | 8 |
| HB1 | 22.555475235 | 25.378625870 | 69.716011047 | CYSn | 40 | h | 0.1000 | 9 |
| HB2 | 23.865217209 | 24.277284622 | 69.317871094 | CYSn | 40 | h | 0.1000 | 10 |
| SG | 23.613842010 | 24.466741562 | 71.679084778 | CYSn | 40 | si | 0.1000 | 11 |
| N | 26.910152435 | 25.881416321 | 70.350471497 | SER | 41 | n | -0.5000 | 12 |
| CA | 28.052598953 | 25.721015930 | 71.310394287 | SER | 41 | ca | 0.1200 | 13 |
| HN | 26.921674728 | 25.473436356 | 69.412322998 | SER | 41 | hn | 0.2800 | 14 |
| HA | 27.761577606 | 24.886217117 | 71.978584290 | SER | 41 | h | 0.1000 | 15 |
| C | 28.477056503 | 26.929338455 | 72.226699829 | SER | 41 | c' | 0.3800 | 16 |
| O | 28.412267685 | 28.097608566 | 71.829902649 | SER | 41 | o' | -0.3800 | 17 |
| CB | 29.257335663 | 25.212779999 | 70.480110168 | SER | 41 | c2 | -0.1700 | 18 |
| HB1 | 28.957101822 | 24.401222229 | 69.786743164 | SER | 41 | h | 0.1000 | 19 |
| HB2 | 29.657680511 | 26.030597687 | 69.845893860 | SER | 41 | h | 0.1000 | 20 |
| OG | 30.284614835 | 24.713315964 | 71.339111328 | SER | 41 | oh | -0.3800 | 21 |
| HG | 31.027490616 | 24.448732376 | 70.785003662 | SER | 41 | ho | 0.3500 | 22 |
| N | 28.904022217 | 26.617259979 | 73.466476440 | GLY | 42 | n | -0.5000 | 23 |
| CA | 29.226711273 | 27.639400482 | 74.497131348 | GLY | 42 | cg | 0.0200 | 24 |
| HN | 29.140472412 | 25.626163483 | 73.587532043 | GLY | 42 | hn | 0.2800 | 25 |
| HA1 | 28.567264557 | 28.519987106 | 74.394309998 | GLY | 42 | h | 0.1000 | 26 |
| HA2 | 28.949186325 | 27.229663849 | 75.483924866 | GLY | 42 | h | 0.1000 | 27 |
| C | 30.728670120 | 28.040773392 | 74.587509155 | GLY | 42 | c' | 0.3800 | 28 |
| O | 31.522300720 | 27.171119690 | 74.961143494 | GLY | 42 | o' | -0.3800 | 29 |
| N | 31.175983429 | 29.296203613 | 74.282577515 | PRO | 43 | n | -0.4200 | 30 |
| CA | 32.627410889 | 29.612504959 | 74.140579224 | PRO | 43 | ca | 0.0600 | 31 |
| HA | 33.153442383 | 28.731788635 | 73.723632813 | PRO | 43 | h | 0.1000 | 32 |
| CD | 30.295356750 | 30.374078751 | 73.784042358 | PRO | 43 | c2 | 0.0600 | 33 |
| HD1 | 29.605636597 | 30.735929489 | 74.571166992 | PRO | 43 | h | 0.1000 | 34 |
| HD2 | 29.683467865 | 30.026647568 | 72.928161621 | PRO | 43 | h | 0.1000 | 35 |
| C | 33.389945984 | 30.112844467 | 75.429031372 | PRO | 43 | c' | 0.3800 | 36 |
| O | 32.754360199 | 30.681560516 | 76.327354431 | PRO | 43 | o' | -0.3800 | 37 |
| CB | 32.565906525 | 30.710325241 | 73.057388306 | PRO | 43 | c2 | -0.2000 | 38 |
| HB1 | 33.449081421 | 31.378034592 | 73.055488586 | PRO | 43 | h | 0.1000 | 39 |
| HB2 | 32.524932861 | 30.247560501 | 72.051818848 | PRO | 43 | h | 0.1000 | 40 |
| CG | 31.263490677 | 31.466632843 | 73.332626343 | PRO | 43 | c2 | -0.2000 | 41 |
| HG1 | 31.413110733 | 32.204200745 | 74.146759033 | PRO | 43 | h | 0.1000 | 42 |
| HG2 | 30.894020081 | 32.022109985 | 72.450286865 | PRO | 43 | h | 0.1000 | 43 |
| N | 34.754648480 | 30.015562057 | 75.523460388 | PRO | 44 | n | -0.4200 | 44 |
| CA | 35.553565979 | 30.763086319 | 76.536285400 | PRO | 44 | ca | 0.0600 | 45 |
| HA | 35.083564758 | 30.695350647 | 77.536567688 | PRO | 44 | h | 0.1000 | 46 |
| CD | 35.574893951 | 29.135835648 | 74.665214539 | PRO | 44 | c2 | 0.0600 | 47 |
| HD1 | 35.471595764 | 29.373666763 | 73.588935852 | PRO | 44 | h | 0.1000 | 48 |
| HD2 | 35.281650543 | 28.076414108 | 74.807395935 | PRO | 44 | h | 0.1000 | 49 |
| C | 35.767509460 | 32.265411377 | 76.141113281 | PRO | 44 | c' | 0.3800 | 50 |
| O | 36.544441223 | 32.599441528 | 75.238464355 | PRO | 44 | o' | -0.3800 | 51 |
| CB | 36.849227905 | 29.927103043 | 76.567779541 | PRO | 44 | c2 | -0.2000 | 52 |
| HB1 | 37.732776642 | 30.502979279 | 76.899009705 | PRO | 44 | h | 0.1000 | 53 |
| HB2 | 36.733722687 | 29.095364598 | 74.269833069 | PRO | 44 | h | 0.1000 | 54 |
| CG | 37.005489349 | 29.369808197 | 75.152616408 | PRO | 44 | c2 | -0.2000 | 55 |
| HG1 | 37.502185822 | 30.119005203 | 74.504158020 | PRO | 44 | h | 0.1000 | 56 |
| HG2 | 37.625408173 | 28.451385498 | 75.115615645 | PRO | 44 | h | 0.1000 | 57 |
| N | 35.047088623 | 33.173435211 | 76.816978455 | ALA | 45 | n | -0.5000 | 58 |
| CA | 35.011333466 | 34.609920502 | 76.449218750 | ALA | 45 | ca | 0.1200 | 59 |
| HN | 34.471405029 | 32.798248291 | 77.590728760 | ALA | 45 | hn | 0.2800 | 60 |
| HA | 35.065380096 | 34.699813843 | 75.343650818 | ALA | 45 | h | 0.1000 | 61 |
| C | 36.187728882 | 35.414947510 | 77.090148926 | ALA | 45 | c' | 0.3800 | 62 |
| O | 36.133388519 | 35.819305420 | 78.255142212 | ALA | 45 | o' | -0.3800 | 63 |
| CB | 33.615478516 | 35.135440826 | 76.831176758 | ALA | 45 | c3 | -0.3000 | 64 |
| HB1 | 33.490375519 | 36.187480927 | 76.517280579 | ALA | 45 | h | 0.1000 | 65 |
| HB2 | 32.811222076 | 34.556259155 | 76.338432312 | ALA | 45 | h | 0.1000 | 66 |
| HB3 | 33.433517456 | 35.098365784 | 77.922439575 | ALA | 45 | h | 0.1000 | 67 |
| N | 37.264499664 | 35.613868713 | 76.306388855 | ALA | 46 | n | -0.5000 | 68 |
| HN | 37.248832703 | 35.072978973 | 75.433502197 | ALA | 46 | hn | 0.2800 | 69 |
| CA | 38.503662109 | 36.298694611 | 76.764076233 | ALA | 46 | ca | 0.1200 | 70 |
| HA | 38.303600311 | 36.883266449 | 77.688095093 | ALA | 46 | h | 0.1000 | 71 |
| C | 39.082061768 | 37.273509979 | 75.687866211 | ALA | 46 | c' | 0.3800 | 72 |
| O | 38.951850891 | 37.052509308 | 74.481193542 | ALA | 46 | o' | -0.3800 | 73 |
| CB | 39.509185791 | 35.179004669 | 77.103065491 | ALA | 46 | c3 | -0.3000 | 74 |
| HB1 | 40.441535950 | 35.582756042 | 77.535072327 | ALA | 46 | h | 0.1000 | 75 |
| HB2 | 39.106670380 | 34.460605621 | 77.839447021 | ALA | 46 | h | 0.1000 | 76 |
| HB3 | 39.780502319 | 34.597728729 | 76.205062866 | ALA | 46 | h | 0.1000 | 77 |
| N | 39.768814087 | 38.344066620 | 76.133750916 | ALA | 47 | n | -0.5000 | 78 |
| CA | 40.322643280 | 39.391151428 | 75.225708008 | ALA | 47 | ca | 0.1200 | 79 |
| HN | 39.783836365 | 38.455593109 | 77.149337769 | ALA | 47 | hn | 0.2800 | 80 |
| HA | 39.932807922 | 39.265201569 | 74.196365356 | ALA | 47 | h | 0.1000 | 81 |
| C | 41.900882721 | 39.401374817 | 75.126907349 | ALA | 47 | c' | 0.3800 | 82 |
| O | 42.538444519 | 40.171451569 | 75.854652405 | ALA | 47 | o' | -0.3800 | 83 |
| CB | 39.728843689 | 40.731719971 | 75.714279175 | ALA | 47 | c3 | -0.3000 | 84 |
| HB1 | 40.043342590 | 41.567916870 | 75.059875488 | ALA | 47 | h | 0.1000 | 85 |
| HB2 | 38.621978760 | 40.726497650 | 75.711242676 | ALA | 47 | h | 0.1000 | 86 |
| HB3 | 40.062076569 | 40.987442017 | 76.739311218 | ALA | 47 | h | 0.1000 | 87 |
| N | 42.578651428 | 38.603939056 | 74.242019653 | PRO | 48 | n | -0.4200 | 88 |
| CA | 44.052474976 | 38.702857971 | 74.013595581 | PRO | 48 | ca | 0.0600 | 89 |
| HA | 44.576034546 | 38.850185394 | 74.977058411 | PRO | 48 | h | 0.1000 | 90 |
| CD | 41.956359863 | 37.474979401 | 73.520225525 | PRO | 48 | c2 | 0.0600 | 91 |
| HD1 | 41.114963531 | 37.788272858 | 72.872795105 | PRO | 48 | h | 0.1000 | 92 |
| HD2 | 41.576156616 | 36.723918915 | 74.239135742 | PRO | 48 | h | 0.1000 | 93 |
| c | 44.492458344 | 39.820354462 | 73.002609253 | PRO | 48 | c' | 0.3800 | 94 |
| O | 43.782276154 | 40.131282806 | 72.040626526 | PRO | 48 | o' | -0.3800 | 95 |
| CB | 44.356296539 | 37.289741516 | 73.479736328 | PRO | 48 | c2 | -0.2000 | 96 |
| HB1 | 45.273612976 | 37.234390259 | 72.865592957 | PRO | 48 | h | 0.1000 | 97 |
| HB2 | 44.513816833 | 36.591526031 | 74.322021484 | PRO | 48 | h | 0.1000 | 98 |
| CG | 43.102409363 | 36.884414673 | 72.700988770 | PRO | 48 | c2 | -0.2000 | 99 |
| HG1 | 43.119277954 | 37.331111908 | 71.685241699 | PRO | 48 | h | 0.1000 | 100 |
| HG2 | 43.010280609 | 35.788948059 | 72.572326660 | PRO | 48 | h | 0.1000 | 101 |
| N | 45.709655762 | 40.366821289 | 73.185493469 | GLY | 49 | n | -0.5000 | 102 |
| CA | 46.317604065 | 41.332912445 | 72.214889526 | GLY | 49 | cg | 0.0200 | 103 |
| HN | 46.169986725 | 40.089691162 | 74.058357239 | GLY | 49 | hn | 0.2800 | 104 |
| HA1 | 45.654991150 | 41.537052155 | 71.351181030 | GLY | 49 | h | 0.1000 | 105 |
| HA2 | 46.406318665 | 42.313266754 | 72.719123840 | CLY | 49 | h | 0.1000 | 106 |
| C | 47.710880280 | 40.963481903 | 71.654037476 | CLY | 49 | c' | 0.380 | 107 |
| O | 48.630664825 | 41.772521973 | 71.754951477 | GLY | 49 | o' | -0.3800 | 108 |
| N | 47.830738068 | 39.763301849 | 71.063682556 | HIS | 50 | n | -0.5000 | 109 |
| HN | 46.918842316 | 39.310573578 | 70.943237305 | HIS | 50 | hn | 0.2800 | 110 |
| CA | 49.045799255 | 39.210880280 | 70.375061035 | HIS | 50 | ca | 0.1200 | 111 |
| HA | 49.315334320 | 38.320884705 | 70.972137451 | HIS | 50 | h | 0.1000 | 112 |
| C | 50.433021545 | 39.981941223 | 70.267852783 | HIS | 50 | c' | 0.3800 | 113 |
| O | 50.773132324 | 40.456230164 | 69.178672791 | HIS | 50 | o' | -0.3800 | 114 |
| CB | 48.558776855 | 38.590164185 | 69.024101257 | HIS | 50 | c2 | -0.2000 | 115 |
| HB1 | 49.390335083 | 33.009521484 | 68.577232361 | HIS | 50 | h | 0.1000 | 116 |
| HB2 | 47.792594910 | 37.817192078 | 69.227310181 | HIS | 50 | h | 0.1000 | 117 |
| CG | 47.997627258 | 39.545143127 | 67.956726074 | HIS | 50 | c5 | 0.1000 | 118 |
| ND1 | 46.669281006 | 39.956676483 | 67.916121338 | HIS | 50 | np | -0.4200 | 119 |
| CE1 | 46.730144501 | 40.789539337 | 66.829002380 | HIS | 50 | c5 | 0.2700 | 120 |
| NE2 | 47.911670685 | 40.950614929 | 66.152328491 | HIS | 50 | np | -0.5000 | 121 |
| CD2 | 48.729324341 | 40.126430511 | 66.904235840 | HIS | 50 | c5 | 0.0100 | 122 |
| HE1 | 45.843067169 | 41.327060699 | 66.517631531 | HIS | 50 | h | 0.1300 | 123 |
| HE2 | 48.138290405 | 41.548683167 | 65.349815369 | HIS | 50 | hn | 0.2800 | 124 |
| HD2 | 49.789726257 | 39.981491089 | 66.738136292 | HIS | 50 | h | 0.1300 | 125 |
| N | 51.307849884 | 40.071182251 | 71.317932129 | PRO | 51 | n | -0.4200 | 126 |
| CA | 52.692558289 | 40.596851349 | 71.184913635 | PRO | 51 | ca | 0.0600 | 127 |
| HA | 52.742668152 | 41.390510559 | 70.412712097 | PRO | 51 | h | 0.1000 | 128 |
| CD | 50.980678558 | 39.703777313 | 72.706970215 | PRO | 51 | c2 | 0.0600 | 129 |
| HD1 | 50.998199463 | 38.605384827 | 72.818397522 | PRO | 51 | h | 0.1000 | 130 |
| HD2 | 49.987606049 | 40.071315765 | 73.019950867 | PRO | 51 | h | 0.1000 | 131 |
| C | 53.739063263 | 39.471630096 | 70.880722046 | PRO | 51 | c' | 0.3800 | 132 |
| O | 53.708900452 | 38.394466400 | 71.488830566 | PRO | 51 | o' | -0.3800 | 133 |
| CB | 52.868911743 | 41.240936279 | 72.572486877 | PRO | 51 | c2 | -0.2000 | 134 |
| HB1 | 53.929355621 | 41.364253998 | 72.864852905 | PRO | 51 | h | 0.1000 | 135 |
| HB2 | 52.429229736 | 42.258647919 | 72.565872192 | PRO | 51 | h | 0.1000 | 136 |
| CG | 52.087848663 | 40.349472046 | 73.547019958 | PRO | 51 | c2 | -0.2000 | 137 |
| HG1 | 52.750400543 | 39.566276550 | 73.963310242 | PRO | 51 | h | 0.1000 | 138 |
| HG2 | 51.686916351 | 40.923263550 | 74.403717041 | PRO | 51 | h | 0.1000 | 139 |
| N | 54.676445007 | 39.726749420 | 69.946899414 | LEU | 52 | n | -0.5000 | 140 |
| CA | 55.768096924 | 38.764469147 | 69.570259094 | LEU | 52 | ca | 0.1200 | 141 |
| HN | 54.573589325 | 40.637012482 | 69.488586426 | LEU | 52 | hn | 0.2800 | 142 |
| HA | 56.414031982 | 39.325927734 | 68.869346619 | LEU | 52 | h | 0.1000 | 143 |
| C | 55.281269073 | 37.540004730 | 68.718757629 | LEU | 52 | c' | 0.3800 | 144 |
| O | 55.654800415 | 37.411125183 | 67.550910950 | LEU | 52 | o' | -0.3800 | 145 |
| CB | 56.713882446 | 38.354763031 | 70.751991272 | LEU | 52 | c2 | -0.2000 | 146 |
| HB1 | 56.125553131 | 37.737205505 | 71.456863403 | LEU | 52 | h | 0.1000 | 147 |
| HB2 | 57.488136292 | 37.658962250 | 70.374801636 | LEU | 52 | h | 0.1000 | 148 |
| CG | 57.411731720 | 39.487998962 | 71.552589417 | LEU | 52 | c1 | -0.1000 | 149 |
| HG | 56.652648926 | 40.244640350 | 71.834617615 | LEU | 52 | h | 0.1000 | 150 |
| CD1 | 58.010108948 | 38.936943054 | 72.859535217 | LEU | 52 | c3 | -0.3000 | 151 |
| HD11 | 58.475826263 | 39.735752106 | 73.467353821 | LEU | 52 | h | 0.1000 | 152 |
| HD12 | 57.236072540 | 38.469894409 | 73.497505188 | LEU | 52 | h | 0.1000 | 153 |
| HD13 | 58.787303925 | 38.171112061 | 72.675750732 | LEU | 52 | h | 0.1000 | 154 |
| CD2 | 58.517623901 | 40.187110901 | 70.742630005 | LEU | 52 | c3 | -0.3000 | 155 |
| HD21 | 58.993679047 | 41.001243591 | 71.321037292 | LEU | 52 | h | 0.1000 | 156 |
| HD22 | 59.321170436 | 39.487018585 | 70.445312500 | LEU | 52 | h | 0.1000 | 157 |
| HD23 | 58.125030518 | 40.647811890 | 69.818283081 | LEU | 52 | h | 0.1000 | 158 |
| N | 54.475013733 | 36.643035889 | 69.315246582 | ALA | 53 | n | -0.5000 | 159 |
| CA | 53.896503448 | 35.451416016 | 68.639259338 | ALA | 53 | ca | 0.1200 | 160 |
| HN | 54.100524902 | 37.007503510 | 70.205230713 | ALA | 53 | hn | 0.2800 | 161 |
| HA | 53.553531647 | 35.773445129 | 67.634338379 | ALA | 53 | h | 0.1000 | 162 |
| C | 52.602260590 | 34.908508301 | 69.353744507 | ALA | 53 | c' | 0.3800 | 163 |
| O | 51.589202881 | 34.828308105 | 68.650024414 | ALA | 53 | o' | -0.3800 | 164 |
| CB | 54.970031738 | 34.364234924 | 68.394615173 | ALA | 53 | c3 | -0.3000 | 165 |
| HB1 | 54.534632636 | 33.449993134 | 67.949813843 | ALA | 53 | h | 0.1000 | 166 |
| HB2 | 55.742454529 | 34.720954895 | 67.688003540 | ALA | 53 | h | 0.1000 | 167 |
| HB3 | 55.500236511 | 34.068145752 | 69.316299438 | ALA | 53 | h | 0.1000 | 168 |
| N | 52.528438568 | 34.519321442 | 70.670852661 | PRO | 54 | n | -0.4200 | 169 |
| CA | 51.288402557 | 33.944232941 | 71.268875122 | PRO | 54 | ca | 0.0600 | 170 |
| HA | 50.860397339 | 33.194515228 | 70.573081970 | PRO | 54 | h | 0.1000 | 171 |
| CD | 53.678298950 | 34.513732910 | 71.601814270 | PRO | 54 | c2 | 0.0600 | 172 |
| HD1 | 54.146690369 | 35.509193420 | 71.717323303 | PRO | 54 | h | 0.1000 | 173 |
| HD2 | 54.456859589 | 33.804187775 | 71.264945984 | PRO | 54 | h | 0.1000 | 174 |
| C | 50.163700104 | 34.973747253 | 71.631500244 | PRO | 54 | c' | 0.3800 | 175 |
| O | 50.381851196 | 36.186935425 | 71.709472650 | PRO | 54 | o' | -0.3800 | 178 |
| CB | 51.868888855 | 33.209735870 | 72.497810364 | PRO | 54 | c2 | -0.2000 | 177 |
| HB1 | 51.140216827 | 33.071922302 | 73.319641113 | PRO | 54 | h | 0.1000 | 178 |
| HB2 | 52.201725006 | 32.193626404 | 72.207275391 | PRO | 54 | h | 0.1000 | 179 |
| CG | 53.074722290 | 34.047100067 | 72.925216675 | PRO | 54 | c2 | -0.2000 | 180 |
| HC1 | 52.742275238 | 34.920612335 | 73.520271301 | PRO | 54 | h | 0.1000 | 181 |
| HG2 | 53.794158936 | 33.482940674 | 73.547462463 | PRO | 54 | h | 0.1000 | 182 |
| N | 48.950717926 | 34.451553345 | 71.882225037 | GLY | 55 | n | -0.5000 | 183 |
| CA | 47.799011230 | 35.264900208 | 72.354187012 | GLY | 55 | cg | 0.0200 | 184 |
| HN | 48.913242340 | 33.427757263 | 71.833122253 | GLY | 55 | hn | 0.2800 | 185 |
| HA1 | 47.829734802 | 36.291049957 | 71.943481445 | GLY | 55 | h | 0.1000 | 186 |
| HA2 | 46.873668671 | 34.838825226 | 71.925086975 | GLY | 55 | h | 0.1000 | 187 |
| C | 47.624210358 | 35.262092590 | 73.898422241 | GLY | 55 | c' | 0.3800 | 188 |
| O | 47.184692383 | 34.228916168 | 74.411125183 | GLY | 55 | o' | -0.3800 | 189 |
| N | 47.910079956 | 36.345748901 | 74.679351807 | PRO | 56 | n | -0.4200 | 190 |
| CA | 47.789894104 | 36.319248199 | 76.162750244 | PRO | 56 | ca | 0.0600 | 191 |
| HA | 48.177116394 | 35.361667633 | 76.567420959 | PRO | 56 | h | 0.1000 | 192 |
| CD | 48.602729797 | 37.547939301 | 74.184982300 | PRO | 56 | c2 | 0.0600 | 193 |
| HD1 | 48.046642303 | 38.065422058 | 73.380996704 | PRO | 56 | h | 0.1000 | 194 |
| HD2 | 49.595470428 | 37.261718750 | 73.794586182 | PRO | 56 | h | 0.1000 | 195 |
| C | 46.326736450 | 36.529109955 | 76.663719177 | PRO | 56 | c' | 0.3800 | 196 |
| O | 45.857757568 | 37.657508850 | 76.842826843 | PRO | 56 | o' | -0.3800 | 197 |
| CB | 48.777050018 | 37.430667877 | 76.578651428 | PRO | 56 | c2 | -0.2000 | 198 |
| HB1 | 48.524734497 | 37.898471832 | 77.549873352 | PRO | 56 | h | 0.1000 | 199 |
| HB2 | 49.795513153 | 37.009433746 | 76.691307068 | PRO | 56 | h | 0.1000 | 200 |
| CG | 48.752750397 | 38.431644440 | 75.422836304 | PRO | 56 | c2 | -0.2000 | 201 |
| HG1 | 47.879917145 | 39.105598450 | 75.522354126 | PRO | 56 | h | 0.1000 | 202 |
| HG2 | 49.655212402 | 39.069591522 | 75.391311646 | PRO | 56 | h | 0.1000 | 203 |
| N | 45.616943359 | 35.415058136 | 76.897827148 | HIS | 57 | n | -0.5000 | 204 |
| HN | 46.014194489 | 34.579135895 | 76.450401306 | HIS | 57 | hn | 0.2800 | 205 |
| CA | 44.212440491 | 35.434158325 | 77.393867493 | HIS | 57 | ca | 0.1200 | 206 |
| HA | 43.635601044 | 36.135776520 | 76.762809753 | HIS | 57 | h | 0.1000 | 207 |
| C | 44.146274567 | 35.882919312 | 78.904235840 | HIS | 57 | c' | 0.3800 | 208 |
| O | 44.718753815 | 35.174930573 | 79.742973328 | HIS | 57 | o' | -0.3800 | 209 |
| CB | 43.577262878 | 34.025093079 | 77.198188782 | HIS | 57 | c2 | -0.2000 | 210 |
| HB1 | 44.242053986 | 33.250709534 | 77.626487732 | HIS | 57 | h | 0.1000 | 211 |
| HB2 | 42.665222168 | 33.964206696 | 77.822631836 | HIS | 57 | h | 0.1000 | 212 |
| CG | 43.190551758 | 33.606594086 | 75.770996094 | HIS | 57 | c5 | 0.1000 | 213 |
| ND1 | 44.009815216 | 33.724720001 | 74.654800415 | HIS | 57 | np | -0.4200 | 214 |
| CE1 | 43.220783234 | 33.103317261 | 73.724327087 | HIS | 57 | c5 | 0.2700 | 215 |
| NE2 | 42.000507355 | 32.603866577 | 74.087806702 | HIS | 57 | np | -0.5000 | 216 |
| CD2 | 42.008693695 | 32.921348572 | 75.433784485 | HIS | 57 | c5 | 0.0100 | 217 |
| HE1 | 43.579235077 | 32.995296478 | 72.708923340 | HIS | 57 | h | 0.1300 | 218 |
| HE2 | 41.324546814 | 32.061363220 | 73.538429260 | HIS | 57 | hn | 0.2800 | 219 |
| HD2 | 41.238662720 | 32.638732910 | 76.138023376 | HIS | 57 | h | 0.1300 | 220 |
| N | 43.493415833 | 37.014366150 | 79.314620972 | PRO | 58 | n | -0.4200 | 221 |
| CA | 43.576023102 | 37.521991730 | 80.713310242 | PRO | 58 | ca | 0.0600 | 222 |
| HA | 44.640773773 | 37.575752258 | 81.019523621 | PRO | 58 | h | 0.1000 | 223 |
| CD | 42.828823090 | 37.960189819 | 78.395561218 | PRO | 58 | c2 | 0.0600 | 224 |
| HD1 | 42.088195801 | 37.471439362 | 77.735382080 | PRO | 58 | h | 0.1000 | 225 |
| HD2 | 43.573791504 | 38.467678070 | 77.749885559 | PRO | 58 | h | 0.1000 | 226 |
| C | 42.782455444 | 36.660888672 | 81.747703552 | PRO | 58 | c' | 0.3800 | 227 |
| O | 41.546211243 | 36.662284851 | 81.766304016 | PRO | 58 | o' | -0.3800 | 228 |
| CB | 43.067096710 | 38.972381592 | 80.563407898 | PRO | 58 | c2 | -0.2000 | 229 |
| HB1 | 42.553604126 | 39.355644226 | 81.465919495 | PRO | 58 | h | 0.1000 | 230 |
| HB2 | 43.922630310 | 39.652961731 | 80.382720947 | PRO | 58 | h | 0.1000 | 231 |
| CG | 42.156223297 | 38.958541870 | 79.333923340 | PRO | 58 | c2 | -0.2000 | 232 |
| HG1 | 41.146640778 | 38.599040985 | 79.611282349 | PRO | 58 | h | 0.1000 | 233 |
| HG2 | 42.036239624 | 39.956802368 | 78.871780396 | PRO | 58 | h | 0.1000 | 234 |
| N | 43.511478424 | 35.934528351 | 82.617271423 | ALA | 59 | n | -0.5000 | 235 |
| HN | 44.509765625 | 35.905010223 | 82.386589050 | ALA | 59 | hn | 0.2800 | 236 |
| CA | 42.913761139 | 34.928112030 | 83.544029236 | ALA | 59 | ca | 0.1200 | 237 |
| HA | 42.154701233 | 34.341350555 | 82.987930298 | ALA | 59 | h | 0.1000 | 238 |
| C | 42.181308746 | 35.481468201 | 84.821502686 | ALA | 59 | c' | 0.3800 | 239 |
| O | 42.459964752 | 35.112228394 | 85.965652466 | ALA | 59 | o' | -0.3800 | 240 |
| CB | 44.063701630 | 33.948829651 | 83.858291626 | ALA | 59 | c3 | -0.3000 | 241 |
| HB1 | 43.708641052 | 33.104522705 | 84.478195190 | ALA | 59 | h | 0.1000 | 242 |
| HB2 | 44.502014160 | 33.503505707 | 82.944122314 | ALA | 59 | h | 0.1000 | 243 |
| HB3 | 44.882900238 | 34.433902740 | 84.422813416 | ALA | 59 | h | 0.1000 | 244 |
| N | 41.178901672 | 36.333106995 | 84.577079773 | ALA | 60 | n | -0.5000 | 245 |
| HN | 41.112052917 | 36.562049866 | 83.573013306 | ALA | 60 | hn | 0.2800 | 246 |
| CA | 40.189502716 | 36.803413391 | 85.578857422 | ALA | 60 | ca | 0.1200 | 247 |
| HA | 40.008514404 | 35.981742859 | 86.302818298 | ALA | 60 | h | 0.1000 | 248 |
| C | 38.811019897 | 37.037807465 | 84.860046387 | ALA | 60 | c' | 0.3800 | 249 |
| O | 37.881835938 | 36.301105499 | 85.205276489 | ALA | 60 | o' | -0.3800 | 250 |
| CB | 40.746566772 | 37.985549927 | 86.401313782 | ALA | 60 | c3 | -0.3000 | 251 |
| HB1 | 39.997776031 | 38.372333527 | 87.116531372 | ALA | 60 | h | 0.1000 | 252 |
| HB2 | 41.624504089 | 37.670467377 | 86.996170044 | ALA | 60 | h | 0.1000 | 253 |
| HB3 | 41.079444885 | 38.830841064 | 85.774902344 | ALA | 60 | h | 0.1000 | 254 |
| N | 38.609931946 | 37.922218323 | 83.826889038 | PRO | 61 | n | -0.4200 | 255 |
| CA | 37.398490906 | 37.873073578 | 82.950836182 | PRO | 61 | ca | 0.0600 | 256 |
| HA | 36.485267639 | 37.839759827 | 83.576164246 | PRO | 61 | h | 0.1000 | 257 |
| CD | 39.552070618 | 39.001556396 | 83.460945129 | PRO | 61 | c2 | 0.0600 | 258 |
| HD1 | 40.590957642 | 38.653537750 | 83.311111450 | PRO | 61 | h | 0.1000 | 259 |
| HD2 | 39.564563751 | 39.780746460 | 84.247795105 | PRO | 61 | h | 0.1000 | 260 |
| C | 37.256782532 | 36.700027466 | 81.909835815 | PRO | 61 | c' | 0.3800 | 261 |
| O | 36.243316650 | 36.657413483 | 81.209106445 | PRO | 61 | o' | -0.3800 | 262 |
| CB | 37.477272034 | 39.256717682 | 82.271400452 | PRO | 61 | c2 | -0.2000 | 263 |
| HB1 | 36.964195251 | 39.298637390 | 81.290786743 | PRO | 61 | h | 0.1000 | 264 |
| HB2 | 36.981357574 | 40.016944885 | 82.906776428 | PRO | 61 | h | 0.1000 | 265 |
| CG | 38.972518921 | 39.562160492 | 82.163795471 | PRO | 61 | c2 | -0.2000 | 266 |
| HG1 | 39.409160614 | 39.034259796 | 81.293624878 | PRO | 61 | h | 0.1000 | 267 |
| HG2 | 39.183399200 | 40.640045166 | 82.032295227 | PRO | 61 | h | 0.1000 | 268 |
| N | 38.213741302 | 35.753643036 | 81.804443359 | SER | 62 | n | -0.5000 | 269 |
| CA | 38.144962311 | 34.600208282 | 80.863403320 | SER | 62 | ca | 0.1200 | 270 |
| HN | 39.009967804 | 35.895751953 | 82.434890747 | SER | 62 | hn | 0.2800 | 271 |
| HA | 37.892318726 | 34.983673096 | 79.856529236 | SER | 62 | h | 0.1000 | 272 |
| C | 37.085021973 | 33.524326324 | 81.273231506 | SER | 62 | c' | 0.3800 | 273 |
| O | 37.382484436 | 32.625560760 | 82.070343018 | SER | 62' | o' | -0.3800 | 274 |
| CB | 39.569152832 | 33.994293213 | 80.760513306 | SER | 62 | c2 | -0.1700 | 275 |
| HB1 | 39.601100922 | 33.242229462 | 79.949050903 | SER | 62 | h | 0.1000 | 276 |
| HB2 | 40.317050934 | 34.759819031 | 80.475799561 | SER | 62 | h | 0.1000 | 277 |
| OG | 39.958038330 | 33.367904663 | 81.986091614 | SER | 62 | h | -0.3800 | 278 |
| HG | 39.157264709 | 32.928077698 | 82.316406250 | SER | 62 | oh | 0.3500 | 279 |
| N | 35.853912354 | 33.643447876 | 80.748901367 | SER | 63 | n | -0.5000 | 280 |
| CA | 34.681579590 | 32.893772125 | 81.280097961 | SER | 63 | ca | 0.1200 | 281 |
| HN | 35.734226227 | 34.507610321 | 80.200576782 | SER | 63 | hn | 0.2800 | 282 |
| HA | 34.978878021 | 32.281963348 | 82.158424377 | SER | 63 | h | 0.1000 | 283 |
| C | 34.028987885 | 31.963005066 | 80.214485168 | SER | 63 | c' | 0.3800 | 284 |
| O | 33.624385834 | 32.404701233 | 79.134857178 | SER | 63 | o' | -0.3800 | 285 |
| CB | 33.674541473 | 33.937458038 | 81.815757751 | SER | 63 | c2 | -0.1700 | 286 |
| HB1 | 34.172107697 | 34.614356995 | 82.538948059 | SER | 63 | h | 0.1000 | 287 |
| HB2 | 33.303077698 | 34.594402313 | 81.003784180 | SER | 63 | h | 0.1000 | 288 |
| OG | 32.576236725 | 33.301517487 | 82.471984863 | SER | 63 | oh | -0.3800 | 289 |
| HG | 32.084590912 | 32.806625366 | 81.807708740 | SER | 63 | ho | 0.3500 | 290 |
| N | 33.852622986 | 30.677625656 | 80.556045532 | TRP | 64 | n | -0.5000 | 291 |
| CA | 33.070865631 | 29.709415436 | 79.732810974 | TRP | 64 | ca | 0.1200 | 292 |
| HN | 34.153343201 | 30.435497284 | 81.506057739 | TRP | 64 | hn | 0.2800 | 293 |
| HA | 33.375720978 | 29.840501785 | 78.676765442 | TRP | 64 | h | 0.1000 | 294 |
| C | 31.526346207 | 29.958730698 | 79.816452026 | TRP | 64 | c' | 0.3800 | 295 |
| O | 30.936735153 | 29.911306381 | 80.900970459 | TRP | 64 | o' | -0.3800 | 296 |
| CB | 33.498836517 | 28.253648758 | 80.086425781 | TRP | 64 | c2 | -0.2000 | 297 |
| HB1 | 32.936897276 | 27.550512314 | 79.442245483 | TRP | 64 | h | 0.1000 | 298 |
| HB2 | 34.548812866 | 28.110004425 | 79.767990112 | TRP | 64 | h | 0.1000 | 299 |
| CG | 33.372638702 | 27.788063049 | 81.551361084 | TRP | 64 | c5 | 0.0000 | 300 |
| CD1 | 32.236022949 | 27.198472977 | 82.145393372 | TRP | 64 | c5 | 0.0100 | 301 |
| NE1 | 32.442039490 | 26.926628113 | 83.513259888 | TRP | 64 | np | -0.5000 | 302 |
| CE2 | 33.734771729 | 27.365074158 | 83.750877380 | TRP | 64 | c5 | 0.1100 | 303 |
| CD2 | 34.313194275 | 27.885219574 | 82.565856934 | TRP | 64 | c5 | 0.0000 | 304 |
| HD1 | 31.308589935 | 27.011001587 | 81.622375488 | TRP | 64 | h | 0.1000 | 305 |
| HE1 | 31.781488419 | 26.532098770 | 84.192108154 | TRP | 64 | hn | 0.2800 | 306 |
| CE3 | 35.633460999 | 28.410655975 | 82.581642151 | TRP | 64 | cp | -0.1000 | 307 |
| HE3 | 36.086208344 | 28.812973022 | 81.686943054 | TRP | 64 | h | 0.1000 | 308 |
| CZ3 | 36.338203430 | 28.401332855 | 83.786201477 | TRP | 64 | cp | -0.1000 | 309 |
| HZ3 | 37.342418671 | 28.800062180 | 83.816780090 | TRP | 64 | h | 0.1000 | 310 |
| CH2 | 35.766292572 | 27.887487411 | 84.956939697 | TRP | 64 | cp | -0.1000 | 311 |
| HH2 | 36.336753845 | 27.895225525 | 85.875114441 | TRP | 64 | h | 0.1000 | 312 |
| CZ2 | 34.469055176 | 27.367534637 | 84.959693909 | TRP | 64 | cp | -0.1000 | 313 |
| HZ2 | 34.033626556 | 26.978828430 | 85.868453979 | TRP | 64 | h | 0.1000 | 314 |
| N | 30.884126663 | 30.253189087 | 78.672058105 | GLY | 65 | n | -0.5000 | 315 |
| CA | 29.433704376 | 30.582933426 | 78.625785828 | GLY | 65 | cg | 0.0200 | 316 |
| HN | 31.490442276 | 30.324731827 | 77.837860107 | GLY | 65 | hn | 0.2800 | 317 |
| HA1 | 29.049486160 | 30.927183151 | 79.604759216 | GLY | 65 | h | 0.1000 | 318 |
| HA2 | 29.301883698 | 31.462034225 | 77.967575073 | GLY | 65 | h | 0.1000 | 319 |
| C | 28.566276550 | 29.436250687 | 78.049354553 | GLY | 65 | c' | 0.3800 | 320 |
| O | 28.476503372 | 29.383417130 | 76.820671082 | GLY | 65 | o' | -0.3800 | 321 |
| N | 27.919076920 | 28.520057678 | 78.830680847 | PRO | 66 | n | -0.4200 | 322 |
| CA | 27.254581451 | 27.307062149 | 78.266265869 | PRO | 66 | ca | 0.0600 | 323 |
| HA | 28.007204056 | 26.749544144 | 77.674018860 | PRO | 66 | h | 0.1000 | 324 |
| CD | 27.931732178 | 28.547025681 | 80.307373047 | PRO | 66 | c2 | 0.0600 | 325 |
| HD1 | 27.674114227 | 29.536535263 | 80.727989197 | PRO | 66 | h | 0.1000 | 326 |
| HD2 | 28.930458069 | 28.264209747 | 80.693565369 | PRO | 66 | h | 0.1000 | 327 |
| C | 25.991449356 | 27.540506363 | 77.367637634 | PRO | 66 | c' | 0.3800 | 328 |
| O | 25.234470367 | 28.498056412 | 77.550445557 | PRO | 66 | o' | -0.3800 | 329 |
| CB | 26.947065353 | 26.487516403 | 79.540168762 | PRO | 66 | c2 | -0.2000 | 330 |
| HB1 | 26.021696091 | 25.883453369 | 79.467781067 | PRO | 66 | h | 0.1000 | 331 |
| HB2 | 27.765922546 | 25.768106461 | 79.730659485 | PRO | 66 | h | 0.1000 | 332 |
| CG | 26.879997253 | 27.505201340 | 80.680580139 | PRO | 66 | c2 | -0.2000 | 333 |
| HG1 | 25.878761292 | 27.974979401 | 80.712356567 | PRO | 66 | h | 0.1000 | 334 |
| HG2 | 27.057765961 | 27.053478241 | 81.674545288 | PRO | 66 | h | 0.1000 | 335 |
| N | 25.764133453 | 26.624628067 | 76.406608582 | CYS | 67 | n | -0.5000 | 336 |
| CA | 24.578670502 | 26.665664673 | 75.512832642 | CYS | 67 | ca | 0.1200 | 337 |
| HN | 26.474828720 | 25.893449783 | 76.320098877 | CYS | 67 | hn | 0.2800 | 338 |
| HA | 24.437746048 | 27.701400757 | 75.152099609 | CYS | 67 | h | 0.1000 | 339 |
| C | 23.256376266 | 26.130277634 | 76.174392700 | CYS | 67 | c' | 0.3800 | 340 |
| O | 23.219629288 | 24.940492630 | 76.516906738 | CYS | 67 | o' | -0.3800 | 341 |
| CB | 24.900175095 | 25.815908432 | 74.260444641 | CYS | 67 | c2 | -0.3000 | 342 |
| HB1 | 25.807971954 | 26.178848267 | 73.749794006 | CYS | 67 | h | 0.1000 | 343 |
| HB2 | 25.105169296 | 24.761932373 | 74.532623291 | CYS | 67 | h | 0.1000 | 344 |
| SG | 23.472158432 | 25.844451904 | 73.133270264 | CYS | 67 | s1 | 0.1000 | 345 |
| N | 22.124137878 | 26.895683289 | 76.264381409 | PRO | 68 | n | -0.4200 | 346 |
| CA | 20.786550522 | 26.297697067 | 76.529830933 | PRO | 68 | ca | 0.0600 | 347 |
| HA | 20.877141953 | 25.506265640 | 77.300582886 | PRO | 68 | h | 0.1000 | 348 |
| CD | 22.161409378 | 28.364057541 | 76.432929993 | PRO | 68 | c2 | 0.0600 | 349 |
| HD1 | 22.628620148 | 28.901371002 | 75.585960388 | PRO | 68 | h | 0.1000 | 350 |
| HD2 | 22.732339859 | 28.631174088 | 77.345329285 | PRO | 68 | h | 0.1000 | 351 |
| C | 20.190311432 | 25.593938828 | 75.255737305 | PRO | 68 | c' | 0.3800 | 352 |
| O | 20.566764832 | 24.451507568 | 74.984413147 | PRO | 68 | o' | -0.3800 | 353 |
| CB | 20.033475876 | 27.483673096 | 77.173645020 | PRO | 68 | c2 | -0.2000 | 354 |
| HB1 | 18.940057755 | 27.449979782 | 77.017181396 | PRO | 68 | h | 0.1000 | 355 |
| HB2 | 20.183664322 | 27.458950043 | 78.271354675 | PRO | 68 | h | 0.1000 | 356 |
| CG | 20.687761307 | 28.746078491 | 76.604209900 | PRO | 68 | c2 | -0.2000 | 357 |
| HG1 | 20.234010696 | 29.017192841 | 75.632743835 | PRO | 68 | h | 0.1000 | 358 |
| HG2 | 20.559530258 | 29.623554230 | 77.265640259 | PRO | 68 | h | 0.1000 | 359 |
| H | 19.297069550 | 26.226366043 | 74.460205078 | ARG+ | 69 | n | -0.5000 | 360 |
| CA | 18.727945328 | 25.594141006 | 73.229873657 | ARG+ | 69 | ca | 0.1200 | 361 |
| HN | 18.899488449 | 27.074655533 | 74.874603271 | ARG+ | 69 | hn | 0.2800 | 362 |
| HA | 19.468439102 | 24.889890671 | 72.798027039 | ARG+ | 69 | h | 0.1000 | 363 |
| C | 18.426181793 | 26.666866302 | 72.127845764 | ARG+ | 69 | c' | 0.3800 | 364 |
| O | 17.302417755 | 27.170328140 | 72.057907104 | ARG+ | 69 | o' | -0.3800 | 365 |
| CB | 17.487716675 | 24.741283417 | 73.645401001 | ARG+ | 69 | c2 | -0.2000 | 366 |
| HB1 | 17.790594101 | 24.038330078 | 74.447227478 | ARG+ | 69 | h | 0.1100 | 367 |
| HB2 | 16.742151260 | 25.409061432 | 74.119949341 | ARG+ | 69 | h | 0.1100 | 368 |
| CG | 16.806570053 | 23.930654526 | 72.510940552 | ARG+ | 69 | c2 | -0.2000 | 369 |
| HG1 | 16.500089645 | 24.624885559 | 71.702163696 | ARG+ | 69 | h | 0.1300 | 370 |
| HG2 | 17.539186478 | 23.235509872 | 72.053100586 | ARG+ | 69 | h | 0.1300 | 371 |
| CD | 15.574314117 | 23.148860931 | 73.007453918 | ARG+ | 69 | c2 | -0.0900 | 372 |
| HD1 | 15.890284538 | 22.374292374 | 73.738624573 | ARG+ | 69 | h | 0.1300 | 373 |
| HD2 | 14.902976036 | 23.843069077 | 73.554016113 | ARG+ | 69 | h | 0.1300 | 374 |
| NE | 14.865127563 | 22.521680832 | 71.855873108 | ARG+ | 69 | n1 | -0.5000 | 375 |
| HE | 15.293711662 | 22.507183075 | 70.926025391 | ARG+ | 69 | hn | 0.3600 | 376 |
| CZ | 13.645489693 | 21.980854034 | 71.902374268 | ARG+ | 69 | cr | 0.4500 | 377 |
| NH1 | 13.127552986 | 21.522832870 | 70.798370361 | ARG+ | 69 | n2 | -0.5000 | 378 |
| HH11 | 13.689088821 | 21.608518600 | 69.948852539 | ARG+ | 69 | hn | 0.3600 | 379 |
| HH12 | 12.188611031 | 21.122539520 | 70.853851318 | ARG+ | 69 | hn | 0.3600 | 380 |
| NH2 | 12.936479568 | 21.886768341 | 73.000465393 | ARG+ | 69 | n2 | -0.5000 | 381 |
| HH21 | 12.008401871 | 21.462900162 | 72.952354431 | ARG+ | 69 | hn | 0.3600 | 382 |
| HH22 | 13.405644417 | 22.251142502 | 73.831558228 | ARG+ | 69 | hn | 0.3600 | 383 |
| N | 19.430337906 | 26.966600418 | 71.273384094 | ARG+ | 70 | n | -0.5000 | 384 |
| CA | 19.316965103 | 27.784936905 | 70.016807556 | ARG+ | 70 | ca | 0.1200 | 385 |
| HN | 20.317523956 | 26.522159576 | 71.527793884 | ARG+ | 70 | hn | 0.2800 | 386 |
| HA | 19.139877319 | 27.013025284 | 69.241798401 | ARG+ | 70 | h | 0.1000 | 387 |
| C | 20.690151215 | 28.397680283 | 69.573341370 | ARG+ | 70 | c' | 0.3800 | 388 |
| O | 21.267679214 | 27.963806152 | 68.579154968 | ARG+ | 70 | o' | -0.3800 | 389 |
| CB | 18.103752136 | 28.753881454 | 69.796676636 | ARG+ | 70 | c2 | -0.2000 | 390 |
| HB1 | 18.134477615 | 29.133897781 | 68.754875183 | ARG+ | 70 | h | 0.1100 | 391 |
| HB2 | 17.183977127 | 28.135446548 | 69.816154480 | ARG+ | 70 | h | 0.1100 | 392 |
| CG | 17.944366455 | 29.952959061 | 70.767364502 | ARG+ | 70 | c2 | -0.2000 | 393 |
| HG1 | 18.201717377 | 29.630409241 | 71.795295715 | ARG+ | 70 | h | 0.1300 | 394 |
| HG2 | 18.686578751 | 30.737569809 | 70.523498535 | ARG+ | 70 | h | 0.1300 | 395 |
| CD | 16.516407013 | 30.528032303 | 70.757499695 | ARG+ | 70 | c2 | -0.0900 | 396 |
| HD1 | 16.205293655 | 30.812314987 | 69.732498169 | ARG+ | 70 | h | 0.1300 | 397 |
| HD2 | 15.804359436 | 29.724859238 | 71.041206360 | ARG+ | 70 | h | 0.1300 | 398 |
| NE | 16.396289825 | 31.632083893 | 71.751823425 | ARG+ | 70 | n1 | -0.5000 | 399 |
| HE | 16.381929398 | 31.418577194 | 72.754409790 | ARG+ | 70 | hn | 0.3600 | 400 |
| CZ | 16.270032883 | 32.931114197 | 71.475357056 | ARG+ | 70 | cr | 0.4500 | 401 |
| NH1 | 16.119342804 | 33.758121490 | 72.470024109 | ARG+ | 70 | n2 | -0.5000 | 402 |
| HH11 | 16.097789764 | 33.352241516 | 73.407394409 | ARG+ | 70 | hn | 0.3600 | 403 |
| HH12 | 16.020824432 | 34.751869202 | 72.242614746 | ARG+ | 70 | hn | 0.3600 | 404 |
| NH2 | 16.295974731 | 33.427280426 | 70.262794495 | ARG+ | 70 | n2 | -0.5000 | 405 |
| HH21 | 16.191591263 | 34.436088562 | 70.142570496 | ARG+ | 70 | hn | 0.3600 | 406 |
| HH22 | 16.439620972 | 32.732715607 | 69.527351379 | ARG+ | 70 | hn | 0.3600 | 407 |
| N | 21.215826035 | 29.506198883 | 70.104598999 | TYRC | 71 | n | -0.5000 | 408 |
| HN | 21.692993164 | 29.961484909 | 69.319816589 | TYRC | 71 | hn | 0.2800 | 409 |
| CA | 22.037544250 | 29.469150543 | 71.348724365 | TYRC | 71 | ca | 0.1200 | 410 |
| BA | 22.727062225 | 28.601654053 | 71.296867371 | TYRC | 71 | h | 0.1000 | 411 |
| C | 21.230804443 | 29.295030594 | 72.663772583 | TYRC | 71 | c' | 0.4100 | 412 |
| OXT | 20.420524597 | 30.105148315 | 73.113685608 | TYRC | 71 | o' | -0.3800 | 413 |
| O | 21.522335052 | 28.107995987 | 73.273979187 | TYRC | 71 | oh | -0.3800 | 414 |
| HO | 20.994127274 | 28.000011444 | 74.066841125 | TYRC | 71 | ho | 0.3500 | 415 |
| CB | 22.938613892 | 30.740638733 | 71.402084351 | TYRC | 71 | c2 | -0.2000 | 416 |
| HB1 | 22.321226120 | 31.652799606 | 71.283157349 | TYRC | 71 | h | 0.1000 | 417 |
| HB2 | 23.363500595 | 30.853305817 | 72.420455933 | TYRC | 71 | h | 0.1000 | 418 |
| CG | 24.110603333 | 30.760416031 | 70.402580261 | TYRC | 71 | cp | 0.0000 | 419 |
| CD1 | 23.933057785 | 31.274461746 | 69.111869812 | TYRC | 71 | cp | -0.1000 | 420 |
| HD1 | 22.977622986 | 31.679159164 | 68.809974670 | TYRC | 71 | h | 0.1000 | 421 |
| CE1 | 24.985538483 | 31.264329910 | 68.201301575 | TYRC | 71 | cp | -0.1000 | 422 |
| HE1 | 24.833002090 | 31.650396347 | 67.203536987 | TYRC | 71 | h | 0.1000 | 423 |
| CZ | 26.227394104 | 30.757091522 | 68.577186584 | TYRC | 71 | cp | 0.0300 | 424 |
| OH | 27.265848160 | 30.762763977 | 67.686424255 | TYRC | 71 | oh | -0.3800 | 425 |
| HH | 26.966634750 | 31.154380798 | 66.863937378 | TYRC | 71 | ho | 0.3500 | 426 |
| CE2 | 26.415199280 | 30.251981735 | 69.859985352 | TYRC | 71 | cp | -0.1000 | 427 |
| HE2 | 27.377700806 | 29.852491379 | 70.147377014 | TYRC | 71 | h | 0.1000 | 428 |
| CD2 | 25.360828400 | 30.253871918 | 70.770927429 | TYRC | 71 | cp | -0.1000 | 429 |
| HD2 | 25.521846771 | 29.846044540 | 71.760574341 | TYRC | 71 | h | 0.1000 | 430 |

| | | | | | | | | |
|---|---|---|---|---|---|---|---|---|
| * La numérotation des aminoacides est décalée de moins 6 par rapport à la séquence SEQ ID N° 1. | | | | | | | | |

## Revendications

1. Utilisation d'anticorps qui stimulent le système immunitaire et qui reconnaissent le domaine extracellulaire D1 de type immunoglobuline de la protéine LAG-3, qui correspond aux acides aminés 1 à 149 de la séquence ID N° 1 ou de fragments de ces anticorps pour la préparation d'une composition thérapeutique destinée au traitement de pathologies qui peuvent être améliorées ou évitées grâce à la stimulation du système immunitaire.

2. Utilisation selon la revendication 1, **caractérisée en ce que** lesdits anticorps sont utilisés comme potentialisateurs de vaccins.

3. Utilisation selon l'une quelconque des revendications 1 ou 2, **caractérisée en ce que** ladite stimulation du système immunitaire correspond à la stimulation des lymphocytes T.

4. Utilisation selon l'une quelconque des revendications 1 à 3, **caractérisée en ce que** ladite stimulation du système immunitaire correspond à la stimulation de la maturation des lymphocytes T.

5. Utilisation selon l'une quelconque des revendications 1 à 4, **caractérisée en ce que** ladite stimulation de la réponse immunitaire correspond à la stimulation de la différenciation des lymphocytes T.

6. Utilisation selon l'une quelconque des revendications 1 à 5, **caractérisée en ce que** ladite stimulation de la réponse immunitaire correspond à la stimulation de la prolifération des lymphocytes T.

7. Utilisation selon l'une quelconque des revendications 1 à 6, **caractérisée en ce que** ladite stimulation de la réponse immunitaire correspond à la stimulation de la fonction des lymphocytes T.

8. Utilisation selon l'une quelconque des revendications 1 à 7, **caractérisée en ce que** les anticorps sont des anticorps monoclonaux ou des fragments de ces anticorps, notamment les fragments Fab, Fab', F(ab')₂.

9. Utilisation selon l'une quelconque des revendications 1 à 8, **caractérisée en ce que** lesdites pathologies sont choisies dans le groupe comprenant les maladies infectieuses et les cancers.

10. Fraction polypeptidique soluble constituée par les 4 domaines extracellulaires de type immunoglobuline de la protéine LAG-3 qui correspond aux acides aminés 1 à 412 de la séquence ID N° 1, **caractérisée en ce que** la séquence peptidique LAG-3 comprend en outre à son extrémité C-terminale et/ou N-terminale une séquence peptidique supplémentaire d'une région jonction -CH2-CH3 d'une chaîne d'immunoglobuline, de manière à constituer une protéine de fusion.

11. Fraction polypeptidique soluble selon la revendication 10, **caractérisée en ce que** l'immunoglobuline est d'isotype IgG4.

## Claims

1. Use of antibodies, which stimulate the immune system and which recognise the extracellular domain D1 of the immunoglobulin type protein LAG-3 corresponding to amino acids 1 to 149 of the ID N° 1 sequence, or fragments of said antibodies for the preparation of a therapeutic composition for the treatment of pathologies, which can be improved or prevented by stimulating of the immune system.

2. Use according to Claim 1, **characterised by** the fact that said antibodies are used as vaccine potentialisers.

3. Use according to Claim 1 or 2, **characterised by** the fact that said stimulation of the immune system corresponds to the stimulation of lymphocytes T.

4. Use according to any one of Claims 1 to 3, **characterised by** the fact that said stimulation of the immune system corresponds to the stimulation of maturing of lymphocytes T.

5. Use according to anyone of Claims 1 to 4, **characterised by** the fact that said stimulation of the immune response corresponds to the stimulation of the differentiation of lymphocytes T.

6. Use according to anyone of Claims 1 to 5, **characterised by** the fact that said stimulation of the immune response corresponds to the stimulation of the proliferation of lymphocytes T.

7. Use according to anyone of Claims 1 to 6, **characterised by** the fact that said stimulation of the immune response corresponds to the stimulation of the function of lymphocytes T.

8. Use according to anyone of Claims 1 to 7, **characterised by** the fact that the antibodies are monoclonal antibodies, or fragments of said antibodies, in particular the fragments Fab, Fab', F(ab')₂

9. Use according to anyone of Claims 1 to 8, **characterised by** the fact that said pathologies are chosen from the group comprising infectious diseases and cancers.

10. A soluble polypeptide fraction comprising the 4 extracellular domains of the immunoglobulin type of protein LAG-3 (corresponding to amino acids 1 to 412 of the ID N° 1 sequence), **characterised by** the fact that the LAG-3 peptide sequence comprises, in addition to its C-terminal and/or N-terminal end, a supplementary peptide sequence of a region junction CH₂-CH₃ of an immunoglobulin chain so as to constitute a fusion protein.

11. A soluble polypeptide fraction from Claim 10, **characterised by** the fact that the immunoglobulin is of the IgG4 isotype

## Patentansprüche

1. Verwendung von Antikörpern, die das Immunsystem stimulieren und die den extrazellulären Bereich D1 vom Typ Immunoglobulin des Proteins LAG-3 erkennen, das die Aminosaüre 1 bis 149 der Sequenz ID Nr. 1 entspricht, oder von Fragmenten dieser Antikörper für die Zubereitung einer therapeutischen Zusammensetzung, die zur Behandlung von Krankheiten bestimmt ist, die durch Stimulation des Immunsystems gelindert oder vermeidbar werden.

2. Verwendung nach Anspruch 1, **dadurch gekennzeichnet, dass** die besagten Antikörper als Impfstoff-Potentialisatoren verwendet werden.

3. Verwendung nach einem der Ansprüche 1 oder 2, **dadurch gekennzeichnet, dass** die besagte Stimulation des Immunsystems der Stimulation der T-Lymphozyten entspricht.

4. Verwendung nach einem der Ansprüche 1 bis 3, **dadurch gekennzeichnet, dass** die besagte Stimulation des Immunsystems der Stimulation der Reifung der T-Lymphozyten entspricht.

5. Verwendung nach einem der Ansprüche 1 bis 4, **dadurch gekennzeichnet, dass** die besagte Stimulation der Immunantwort der Stimulation der Differenzierung der T-Lymphozyten entspricht.

6. Verwendung nach einem der Ansprüche 1 bis 5, **dadurch gekennzeichnet, dass** die besagte Stimulation der Immunantwort der Stimulation der Proliferation der T-Lymphozyten entspricht.

7. Verwendung nach einem der Ansprüche 1 bis 6, **dadurch gekennzeichnet, dass** die besagte Stimulation der Immunantwort der Stimulation der Funktion der T-Lymphozyten entspricht.

8. Verwendung nach einem der Ansprüche 1 bis 7, **dadurch gekennzeichnet, dass** die Antikörper monoklonale Antikörper oder Fragmente dieser Antikörper sind, vor allem die Fragmente Fab, Fab', F(ab')₂.

9. Verwendung nach einem der Ansprüche 1 bis 8, **dadurch gekennzeichnet, dass** die besagten Krankheiten in der Gruppe ausgewählt sind, welche die Infektions- und Krebskrankheiten umfassen.

10. Lösliche Polypeptidfraktion, gebildet von den 4 extrazellulären Bereichen vom Typ Immunoglobulin des Proteins LAG-3, das die Aminosaüre 1 bis 412 der Sequenz ID Nr. 1 entspricht, **dadurch gekennzeichnet, dass** die Peptidsequenz LAG-3 außerdem an ihrem C-terminalen und/oder N-terminalen Ende eine zusätzliche Peptidsequenz einer Verbindungsregion -CH2-CH3 einer Immunoglobulinkette umfasst, so dass ein Fusionsprotein gebildet wird.

11. Lösliche Polypeptidfraktion nach Anspruch 10, **dadurch gekennzeichnet, dass** das Immunoglobulin vom Isotyp IgG4 ist.
